(19) Europäisches Patentamt
European Patent Office
Office européen des brevets

(11) Veröffentlichungsnummer: **0 327 986 A2**

(12) # EUROPÄISCHE PATENTANMELDUNG

(21) Anmeldenummer: 89101868.1

(22) Anmeldetag: 03.02.89

(51) Int. Cl.⁴: C07D 209/40 , C07D 403/12 , C07D 417/12 , C07D 209/96 , C07D 215/38 , C07D 401/12 , C07D 215/50 , C07D 409/12 , C07D 405/12 , A61K 31/40 , A61K 31/415 , //A61K31/425, A61K31/47

(30) Priorität: 09.02.88 DE 3803775

(43) Veröffentlichungstag der Anmeldung: 16.08.89 Patentblatt 89/33

(84) Benannte Vertragsstaaten: AT BE CH DE ES FR GB GR IT LI LU NL SE

(71) Anmelder: BOEHRINGER MANNHEIM GMBH
Sandhofer Strasse 116
D-6800 Mannheim 31(DE)

(72) Erfinder: Zilch, Harald, Dr.rer.nat.
Alsenweg 2 A
D-6800 Mannheim 31(DE)
Erfinder: Mertens, Alfred, Dr.rer.nat.
Beethovenstrasse 20
D-6905 Schriesheim(DE)
Erfinder: Von der Saal, Wolfgang, Dr.rer.nat.
Neuer Burgweg 3
D-6940 Weinheim(DE)
Erfinder: Boehm, Erwin, Dr.med.
Hinterer Rindweg 37
D-6802 Ladenburg(DE)
Erfinder: Strein, Klaus, Prof. Dr. Dr.
Eichenstrasse 45
D-6944 Hemsbach(DE)

(54) **Arzeneimittel enthaltend Lactame, neue substituierte Lactame und Verfahren zu ihrer Herstellung.**

(57) Die vorliegende Erfindung betrifft neue Arzneimittel enthaltend Lactame der Formel I

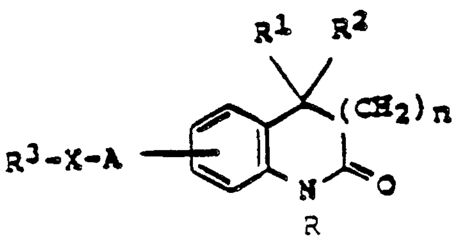

(I),

in welcher
R ein Wasserstoffatom, eine Alkyl- oder Cycloalkylgruppe darstellt,
$R^1$ ein Wasserstoffatom, eine Alkyl-, Alkenyl- oder eine Cycloalkylgruppe bedeutet,
$R^2$ eine Alkyl-, Alkenyl- oder Cyangruppe, eine durch eine Hydroxy-, Alkyl-, Alkoxy-, Amino-, Alkylamino-, Dialkylamino- oder Hydrazinogruppe substituierte Carbonylgruppe bedeutet,
oder $R^1$ und $R^2$ zusammen eine Alkyliden- bzw. Cycloalkylidengruppe darstellen,
oder $R^1$ und $R^2$ zusammen mit dem Kohlenstoffatom, an das sie gebunden sind, einen Spirocyclus bilden,
n gleich 0 oder 1 sein kann,

EP 0 327 986 A2

A in den Substituenten $R^3$-X-A- für die Gruppen $R^3$-X-CO-NH- , $R^3$-X-NH-CO-NH- oder $R^3$-X-O-CO-NH- steht, in denen
X einen Valenzstrich, eine Alkylenkette oder die Vinylgruppe darstellt,
$R_3$ einen ein- oder mehrfach substituierten Phenylring, oder einen heterocyclischen Fünfring mit 1 - 4 Heteroatomen, oder einen Pyrazin-, Pyridazin-, Oxazin-, Thiazin-, Triazin-, Tetrazinring bzw. Chinolinrest, oder für den Fall, daß X eine Bindung darstellt, $R_3$ neben den genannten Resten auch eine $C_2$-$C_{12}$-Alkyl oder Formylaminoalkylgruppen bedeutet, deren Tautomere und deren physiologisch verträgliche Salze.
Gegenstand der vorliegenden Erfindung sind auch neue Lactame der allgemeinen Formel I sowie Verfahren zu ihrer Herstellung und diese enthaltende Arzneimittel zur Behandlung von Krankheiten, bei denen die Erythrozyten- und Thrombozytenaggregation eine wichtige Rolle spielen.

## Arzneimittel enthaltend Lactame, neue substituierte Lactame und Verfahren zu ihrer Herstellung

Die vorliegende Erfindung betrifft neue Arzneimittel, die substituierte Lactame der allgemeinen Formel I

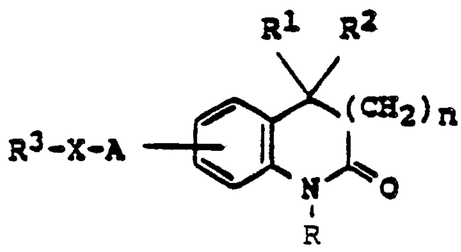

(I),

enthalten,
in welcher
R ein Wasserstoffatom, eine geradkettige oder verzweigte $C_1$-$C_6$-Alkylgruppe oder eine $C_3$-$C_6$-Cycloalkylgruppe darstellt,
$R^1$ ein Wasserstoffatom, eine $C_1$-$C_6$-Alkyl-, $C_2$-$C_6$-Alkenyl-oder eine $C_3$-$C_7$-Cycloalkylgruppe bedeutet,
$R^2$ eine $C_1$-$C_6$-Alkyl-, $C_2$-$C_6$-Alkenyl- oder Cyangruppe, eine durch eine Hydroxy-, $C_1$-$C_6$-Alkyl-, $C_1$-$C_6$-Alkoxy-, Amino-, $C_1$-$C_6$-Alkylamino-, Di-$C_1$-$C_6$-alkylamino- oder Hydrazinogruppe substituierte Carbonylgruppe bedeutet, oder $R^1$ und $R^2$ zusammen eine $C_2$-$C_6$-Alkyliden- bzw. $C_3$-$C_6$-Cycloalkylidengruppe darstellen, oder $R^1$ und $R^2$ zusammen mit dem Kohlenstoffatom, an das sie gebunden sind, einen $C_3$-$C_7$-Spirocyclus bilden,
n gleich 0 oder 1 sein kann,
A die Gruppe -CO-NH-, -NH-CO-NH- oder -O-CO-NH- bedeutet, die über das Stickstoffatom an die Phenylgruppe gebunden ist,
X einen Valenzstrich, eine $C_1$-$C_4$-Alkylengruppe oder eine $C_2$-$C_4$-Alkenylengruppe darstellt,
$R^3$ einen Phenylring der allgemeinen Formel II.

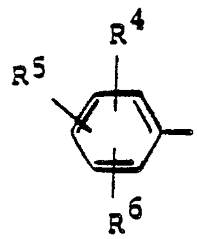

(II)

darstellt, wobei $R^4$, $R^5$, $R^6$ gleich oder verschieden sein können und jeweils Wasserstoff, eine $C_1$-$C_7$-Alkansulfonyloxy-, Trifluormethansulfonyloxy-, $C_1$-$C_7$-Alkansulfonylamino-, Trifluormethansulfonylamino-, N-$C_1$-$C_7$-Alkyl-$C_1$-$C_7$-alkansulfonylamino-, N-$C_1$-$C_7$-Alkyl-trifluormethansulfonylamino-, $C_1$-$C_7$-Alkylsulfenylmethyl-, $C_1$-$C_7$-Alkylsulfinylmethyl- oder $C_1$-$C_7$-Alkylsulfonylmethylgruppe, eine durch eine Hydroxy-, $C_1$-$C_7$-Alkoxy-, $C_1$-$C_7$-Alkyl, Amino-, $C_1$-$C_7$-Alkylamino- oder Di-$C_1$-$C_7$-alkylaminogruppe substituierte Carbonylgruppe, eine durch eine Amino-, $C_1$-$C_7$-Alkylamino-, Di-$C_1$-$C_7$-alkylamino-, Morpholino-, Thiomorpholino-, Pyrrolidino-, Piperidino-oder Hexamethyleniminogruppe substituierte Sulfonylgruppe, eine $C_1$-$C_7$-Alkylcarbonylamino-, $C_1$-$C_7$-Alkylcarbonyloxy-, Aminocarbonylamino-oder $C_1$-$C_7$-Alkylaminocarbonylaminogruppe, eine $C_1$-$C_7$-Alkylmercapto-, $C_1$-$C_7$-Alkylsulfinyl- oder $C_1$-$C_7$-Alkylsulfonylgruppe, eine Nitro-, Amino-, Hydroxy-, Benzyloxy-, $C_1$-$C_7$-Alkoxy-, $C_1$-$C_7$-Alkyl-, $C_2$-$C_7$-Alkenyl-, $C_2$-$C_7$-Alkenyloxy-, $C_2$-$C_7$-Alkinyloxy-, Cyan-$C_1$-$C_7$-alkoxy-, Carboxy-$C_1$-$C_7$-alkoxy-, Phenyl-$C_1$-$C_7$-alkoxy-, $C_1$-$C_7$-Alkoxy-carbonyl-$C_1$-$C_7$-alkoxy-, $C_1$-$C_7$-Alkylamino-, Di-$C_1$-$C_7$-alkylamino-, Trifluormethyl-, Cyano-, Halogen- oder Imidazolylgruppe sein können oder $R_3$ einen Methylendioxyphenylring bedeutet,
oder $R^3$ einen heterocyclischen Fünf- oder Sechsring mit 1-4 bzw. 1-5 Heteroatomen darstellt, wobei die Heteroatome gleich oder verschieden sein können und Sauerstoff, Stickstoff oder Schwefel bedeuten, und die heterocyclischen Fünf- oder Sechsring gewünschtenfalls an einem oder mehreren Stickstoffatomen ein Sauerstoffatom tragen können und gegebenenfalls durch eine oder mehrere $C_1$-$C_6$-Alkyl-, $C_1$-$C_6$-Alkoxy-, $C_1$-$C_6$-Alkylmercapto-, Hydroxy-$C_1$-$C_6$-alkyl, Hydroxy-, Nitro-, Amino-, $C_1$-$C_6$-Alkylamino-, Di-$C_1$-$C_6$-alkyl-

amino-, Halogen- oder Cyanogruppen substituiert sein können, oder für den Fall, daß X eine Bindung darstellt, $R^3$ neben den oben genannten Gruppen auch eine $C_2$-$C_{12}$ Alkyl-, $C_3$-$C_7$-Cycloalkyl-, $C_2$-$C_6$-Alkenyl-, $C_1$-$C_6$-Alkoxy-$C_2$-$C_6$-alkenyl-, $C_3$-$C_7$-Cycloalkenyl-, $C_2$-$C_6$-Alkinyl-, Halogen-$C_1$-$C_6$-alkyl-, $C_1$-$C_6$-Alkoxy-$C_1$-$C_6$-alkyl-, Hydroxy-$C_1$-$C_6$-alkyl-, $C_4$-$C_6$-Alkandienyl-, Carboxy-$C_1$-$C_6$-alkyl-, $C_1$-$C_6$-Alkoxycarbonyl-$C_1$-$C_6$-alkyl-oder Formylamino-$C_1$-$C_6$-alkylgruppe bedeutet, wobei der Substituent $R^3$-X-A- in 4-, 5-, 6-, oder 7-Stellung mit dem 2,3-Dihydroindolin-2-on bzw. in 5-, 6-, 7- oder 8-Stellung mit dem 1,2,3,4-Tetrahydrochinolin-2-on verknüpft sein kann, oder deren optisch aktive Formen, Tautomere oder physiologisch verträglichen Salze.

Gegenstand der vorliegenden Erfindung sind auch neue Verbindungen der allgemeinen Formel I, in der R ein Wasserstoffatom, eine geradkettige oder verzweigte $C_1$-$C_6$-Alkylgruppe oder eine $C_3$-$C_6$-Cycloalkylgruppe darstellt,
$R^1$ ein Wasserstoffatom, eine $C_1$-$C_6$-Alkyl-, $C_2$-$C_6$-Alkenyl-oder eine $C_3$-$C_7$-Cycloalkylgruppe bedeutet,
$R^2$ eine $C_1$-$C_6$-Alkyl-, $C_2$-$C_6$-Alkenyl- oder Cyangruppe, eine durch eine Hydroxy-, $C_1$-$C_6$-Alkyl-, $C_1$-$C_6$-Alkoxy-, Amino-, $C_1$-$C_6$-Alkylamino-, Di-$C_1$-$C_6$-alkylamino- oder Hydrazinogruppe substituierte Carbonylgruppe bedeutet, oder $R^1$ und $R^2$ zusammen eine $C_2$-$C_6$-Alkyliden- bzw. $C_3$-$C_6$-Cycloalkylidengruppe darstellen, oder $R^1$ und $R^2$ zusammen mit dem Kohlenstoffatom, an das sie gebunden sind, einen $C_3$-$C_7$-Spirocyclus bilden,
n gleich 0 oder 1 sein kann,
A die Gruppe -CO-NH-, -NH-CO-NH- oder -O-CO-NH- bedeutet, die über das Stickstoffatom an die Phenylgruppe gebunden ist,
X einen Valenzstrich, eine $C_1$-$C_4$-Alkylengruppe oder eine $C_2$-$C_4$-Alkenylengruppe darstellt,
$R^3$ einen Phenylring der allgemeinen Formel II

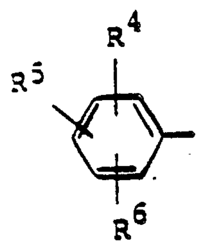

(II) .

darstellt, wobei $R^4$, $R^5$, $R^6$ gleich oder verschieden sein können und jeweils Wasserstoff, eine $C_1$-$C_7$-Alkansulfonyloxy-, Trifluormethansulfonyloxy-, $C_1$-$C_7$-Alkansulfonylamino-, Trifluormethansulfonylamino-, N-$C_1$-$C_7$-Alkyl-$C_1$-$C_7$-alkansulfonylamino-, N-$C_1$-$C_7$-Alkyl-trifluormethansulfonylamino-, $C_1$-$C_7$-Alkylsulfenylmethyl-, $C_1$-$C_7$-Alkylsulfinylmethyl- oder $C_1$-$C_7$-Alkylsulfonylmethylgruppe, eine durch eine Hydroxy-, $C_1$-$C_7$-Alkoxy-, $C_1$-$C_7$-Alkyl, Amino-, $C_1$-$C_7$-Alkylamino- oder Di-$C_1$-$C_7$-alkylaminogruppe substituierte Carbonylgruppe, eine durch eine Amino-, $C_1$-$C_7$-Alkylamino-, Di-$C_1$-$C_7$-alkylamino-, Morpholino-, Thiomorpholino-, Pyrrolidino-, Piperidino- oder Hexamethyleniminogruppe substituierte Sulfonylgruppe, eine $C_1$-$C_7$-Alkylcarbonylamino-, $C_1$-$C_7$-Alkylcarbonyloxy-, Aminocarbonylamino-oder $C_1$-$C_7$-Alkylaminocarbonylaminogruppe, eine $C_1$-$C_7$-Alkylmercapto-, $C_1$-$C_7$-Alkylsulfinyl-oder $C_1$-$C_7$-Alkylsulfonylgruppe, eine Nitro-, Amino-, Hydroxy-, Benzyloxy-, $C_1$-$C_7$-Alkoxy-, $C_1$-$C_7$-Alkyl-, $C_2$-$C_7$-Alkenyl-, $C_2$-$C_7$-Alkenyloxy-, $C_2$-$C_7$-Alkinyloxy-, Cyan-$C_1$-$C_7$-alkoxy-, Carboxy-$C_1$-$C_7$-alkoxy-, Phenyl-$C_1$-$C_7$-alkoxy-, $C_1$-$C_7$-Alkoxy-carbonyl-$C_1$-$C_7$-alkoxy-, $C_1$-$C_7$-Alkylamino-, Di-$C_1$-$C_7$-alkylamino-, Trifluormethyl-, Cyano-, Halogen- oder Imidazolylgruppe sein können oder $R_3$ einen Methylendioxyphenylring bedeutet,
oder $R^3$ einen heterocyclischen Fünf- oder Sechsring mit 1-4 bzw. 1-5 Heteroatomen darstellt, wobei die Heteroatome gleich oder verschieden sein können und Sauerstoff, Stickstoff oder Schwefel bedeuten, und die heterocyclischen Fünf- oder Sechsring gewünschtenfalls an einem oder mehreren Stickstoffatomen ein Sauerstoffatom tragen können und gegebenenfalls durch eine oder mehrere $C_1$-$C_6$-Alkyl-, $C_1$-$C_6$-Alkoxy-, $C_1$-$C_6$-Alkylmercapto-, Hydroxy-$C_1$-$C_6$-alkyl, Hydroxy-, Nitro-, Amino-, $C_1$-$C_6$-Alkylamino-, Di-$C_1$-$C_6$-alkylamino-, Halogen- oder Cyanogruppen substituiert sein können,
oder für den Fall, daß X eine Bindung darstellt, $R^3$ neben den oben genannten Gruppen auch eine $C_2$-$C_{12}$ Alkyl-, $C_3$-$C_7$-Cycloalkyl-, $C_2$-$C_6$-Alkenyl-, $C_1$-$C_6$-Alkoxy-$C_2$-$C_6$-alkenyl-, $C_3$-$C_7$-Cycloalkenyl-, $C_2$-$C_6$-Alkinyl-, Halogen-$C_1$-$C_6$-alkyl-, $C_1$-$C_6$-Alkoxy-$C_1$-$C_6$-alkyl-, Hydroxy-$C_1$-$C_6$-alkyl-, $C_4$-$C_6$-Alkandienyl-, Carboxy-$C_1$-$C_6$-alkyl-, $C_1$-$C_6$-Alkoxycarbonyl-$C_1$-$C_6$-alkyl-oder Formylamino-$C_1$-$C_6$-alkylgruppe bedeutet,
wobei der Substituent $R^3$-X-A- in 4-, 5-, 6-, oder 7-Stellung mit dem 2,3-Dihydroindolin-2-on bzw. in 5-, 6-, 7- oder 8-Stellung mit dem 1,2,3,4-Tetrahydrochinolin-2-on verknüpft sein kann, oder
deren optisch aktive Formen, Tautomere oder physiologisch verträglichen Salze, mit der Maßgabe, daß für

den Fall, daß n die Zahl 0 und A die Gruppe -CO-NH- bedeutet, $R_3$ nicht einen Phenylring der allgemeinen Formel II oder einen heterocyclischen Fünf- oder Sechsring bedeuten kann.

Die Verbindungen können als Stereoisomerengemische oder cis-bzw. trans-Isomere vorliegen.

Für den Fall, daß $R^1$ nicht gleich $R^2$ ist sowie bei Verbindungen mit einem anderen Asymmetriezentrum, sind auch die optisch aktiven Formen und racemischen Gemische der Verbindungen Gegenstand dieser Erfindung.

Einige Verbindungen der allgemeinen Formel I sind als Zwischenprodukte zur Synthese von Pyrrolo-[2,3-f]benzimidazolen bereits literaturbekannt. So sind zum Beispiel in EP-A-0,161,632 Oxindol-Derivate beschrieben, die in 5- oder 6-Stellung durch eine Pyridyl-carbonylamino-Gruppe substituiert sind. Aus EP-A-186,010 sind die entsprechenden Phenyl-carbonylamino-substituierte Derivate bekannt. Ferner enthält EP-A- 189,103 Oxindole, die in 5- oder 6-Stellung eine über eine Carbonylaminogruppe gebundene heterocyclische Gruppe enthalten. Jedoch handelt es sich bei diesen Verbindungen ausschließlich um Zwischenprodukte, für die keine pharmakologische Wirkung beschrieben ist.

Weiterhin sind aus JP 57/102 863 (1982) 6′-Acetylamino-2′-oxospiroindol-Derivate bekannt, die antihypertensive Eigenschaften und plättchenaggregationshemmende Wirkung besitzen.

Ferner ist in der PCT Int. Appl. WO 85/5378 (1985) ein 4-Trifluormethyl-1,2,3,4-tetrahydrochinolin-2-on-Derivat mit enzymspezifischem Substituenten in 6-Stellung zum Nachweis spezifischer Mikroorganismen in Körperflüssigkeiten und in der DE-OS 3 626 465 die Verwendung eines 6-[3-(2-Aryloxybutanoylamino)-benzoylamino]-1,2,3,4-tetrahydrochinolin-2-on-Derivats in colorphotographischen Materialien beschrieben. Hinweise auf eine inotrope Wirkung von 1,2,3,4-Tetrahydrochinolin-2-on-Derivaten mit Substituenten in 6-Stellung finden sich in den Anmeldungen JP 61/145 162 A2 (1986) und EP-OS 145 010, jedoch sind diese Verbindungen in 4-Stellung unsubstituiert.

Es wurde nun überraschenderweise gefunden, daß die Verbindungen der allgemeinen Formel I sowohl die Erythrozytenaggregation als auch die Thrombozytenaggregation in geringen Konzentrationen hemmen. Dies konnte anhand von in-vitro-Untersuchungen belegt werden.

Aufgrund dieser Eigenschaften sind diese Substanzen geeignet zur Behandlung von Krankheiten, bei denen in der Pathogenese die Erythrozyten- und Thrombozytenaggregation eine wichtige Rolle spielen, wie zum Beispiel periphere, coronare und cerebrale Durchblutungsstörungen, Schockzustände, degenerative Gefäßerkrankungen, rheumatische Erkrankungen, verschiedene Arten von Ulcera, nekrotischen Prozessen in Tumoren, degenerative Störungen der Retina, Nerven und Muskeln oder von verschiedenen Hautkrankheiten. Insbesondere kommt die Behandlung von arteriellen Verschlußkrankheiten, ischämischen Zuständen, venöser Insuffizienz oder Diabetes mellitus in Frage. Die Verbindungen der allgemeinen Formel I stellen einen neuen Strukturtyp von Chemotherapeutica dar, wobei diese Chemotherapeutica die ersten Substanzen sind, die in pharmakologisch relevanten Konzentrationen eine Verminderung der Erythrocytenaggregation bewirken und somit rheologisch wirksam sind.

Bedeutet $R^3$ einen Phenylring der allgemeinen Formel II, so kann der Alkylteil der bei $R^4$, $R^5$ und $R^6$ genannten Substituenten 1-7 Kohlenstoffatome enthalten, vorzugsweise 1-4 Kohlenstoffatome. Bevorzugt in diesem Sinne sind beispielsweise die Methansulfonyloxy-, Ethansulfonyloxy-, n-Propansulfonyloxy-, Isopropansulfonyloxy-, Trifluormethansulfonyloxy-, Methylsulfenylmethyl-, Ethylsulfenylmethyl-, n-Propylsulfenylmethyl-, Methylsulfinylmethyl-, Ethylsulfinylmethyl-, n-Propylsulfinylmethyl-, Methylsulfonylmethyl-, Ethylsulfonylmethyl-, n-Propylsulfonylmethyl-, Methansulfonylamino-, Ethansulfonylamino-, n-Propansulfonylamino-, Trifluormethansulfonylamino-, N-Methyl-methansulfonylamino-, N-Ethyl-methansulfonylamino-, N-Methyl-ethansulfonylamino-, N-Ethyl-ethansulfonylamino-, N-Isopropyl-ethansulfonylamino-, N-Methyl-n-propansulfonylamino-, N-n-Propyl-n-propansulfonylamino-, N-Methyl-trifluormethansulfonylamino-, N-Ethyl-tri fluormethansulfonylamino-, N-Isopropyl-trifluormethansulfonylamino-, Methoxycarbonyl-, Ethoxycarbonyl-, Propoxycarbonyl-, Isopropoxycarbonyl-, Methylaminocarbonyl-, Ethylaminocarbonyl-, Dimethylaminocarbonyl, Di-n-propylaminocarbonyl-, N-Methyl-ethylaminocarbonyl-, Trifluormethyl-, Methylaminosulfonyl-, Ethylaminosulfonyl-, n-Propylaminosulfonyl-, n-Butylaminosulfonyl-, n-Pentylaminosulfonyl-, Dimethylaminosulfonyl-, Diethylaminosulfonyl-, Di-n-propylaminosulfonyl-, N-Methyl-isopropylaminosulfonyl-, Acetylamino-, Propionylamino-, Methylcarbonylamino-, Ethylaminocarbonylamino- oder Propylaminocarbonylaminogruppe, eine Methyl-, Ethyl-, Propyl-, Methoxy-, Ethoxy-, Propyloxy-, Allyloxy-, 2-Butenyloxy-, 3-Butenyloxy-, 2-Pentenyloxy-, Propargyloxy-, 2-Butinyloxy-, 3-Butinyloxy-, Cyanmethyloxy-, Cyanethyloxy-, Methoxycarbonylmethyloxy-, Methoxycarbonylethyloxy-, Methylmercapto-, Ethylmercapto-, Methylsulfinyl-, Ethylsulfinyl-, Methylsulfonyl oder die Ethylsulfonylgruppe.

Insbesondere sind bevorzugt

für $R^4$ Wasserstoff, eine Alkylsulfonyloxy-, Trifluormethylsulfonyloxy-, Alkylsulfenylmethyl-, Alkylsulfinylmethyl-, Alkylsulfonylmethyl-, Alkylsulfonylamino-, N-Alkyl-alkylsulfonylamino-, Trifluormethylsulfonylamino- oder N-Alkyltrifluormethylsulfonylaminogruppe, eine durch eine Hydroxy-,

Alkoxy-, Amino-, Alkylamino- oder Dialkylaminogruppe substituierte Carbonylgruppe oder eine durch eine Amino-, Dialkylamino- oder Morpholinogruppe substituierte Sulfonylgruppe, wobei jeder der vorstehend genannten Alkylteile 1 oder 2 Kohlenstoffatome enthalten kann, eine Nitro-, Cyan- oder Alkylaminosulfonylgruppe mit 1-4 Kohlenstoffatomen, eine Alkylcarbonylamino-, Alkylcarbonyloxy-, Aminocarbonylamino-oder N-Alkyl-aminocarbonylaminogruppe, eine Alkylmercapto-, Alkylsulfinyl- oder Alkylsulfonylgruppe, wobei jeder der vorgenannten Alkylteile 1 oder 2 Kohlenstoffatome enthalten kann, eine Amino-, Hydroxy-, Benzyloxy-, Dialkylamino-, Alkyl-, Alkoxy-, Alkenyloxy- oder Alkinyloxygruppe vorzugsweise mit 1-3 Kohlenstoffatomen, eine Arylalkyloxy-, Cyanmethyloxy- oder Methoxycarbonylmethyloxygruppe, die Trifluormethylgruppe, die 1-Imidazolylgruppe oder ein Halogenatom,

für $R^5$ Wasserstoff, eine Hydroxygruppe, eine Alkylgruppe mit 1-3 Kohlenstoffatomen, eine Alkoxy-oder Dialkylaminogruppe mit 1 oder 2 Kohlenstoffatomen in jedem Alkylteil oder ein Halogenatom und

für $R^6$ Wasserstoff oder die Methoxygruppe.

Der Phenylteil kann 1 bis 3 der genannten Substituenten tragen.

Bevorzugte monosubstituierte Phenylverbindungen sind die Hydroxy-, $C_1$-$C_7$ Alkyl-, $C_1$-$C_7$ Alkoxy-, Allyloxy-, Propargyloxy-, Cyanmethyloxy-, Benzyloxy-, Methoxycarbonylmethyloxy-, Halogen-, Nitro-, Cyan-, Aminocarbonyl-, Methoxycarbonyl-, Amino-, Trifluormethyl-, $C_1$-$C_3$ Alkylcarbonyloxy-, $C_1$-$C_3$ Dialkylamino-, $C_1$-$C_3$ Alkylmercapto-, $C_1$-$C_3$ Alkylsulfinyl-, $C_1$-$C_3$ Alkylsulfonyl-, $C_1$-$C_3$ Alkylsulfonyloxy- und die 1-Imidazolyl-phenyle, wobei der Substituent in 2-, 3- oder 4-Stellung stehen kann.

Bevorzugte disubstituierte Phenyle enthalten als Substituenten eine Alkansulfonyloxy-, Trifluormethylsulfonyloxy-, Alkylsulfenylmethyl-, Alkylsulfinylmethyl-, Alkylsulfonylmethyl-, Alkylsulfonylamino-, N-Alkyl-alkylsulfonylamino-, Trifluormethylsulfonylamino- oder N-Alkyl-trifluormethylsulfonylaminogruppe, eine durch eine Hydroxy-, Alkoxy-, Amino-, Alkylamino- oder Dialkylaminogruppe substituierte Carbonylgruppe oder eine durch eine Amino-, Dialkylamino- oder Morpholinogruppe substituierte Sulfonylgruppe, eine Alkylaminosulfonyl-, Alkylcarbonylamino-, Aminocarbonylamino-oder N-Alkyl-aminocarbonylaminogruppe, eine Hydroxy-, Alkyl-, Alkoxy-, Allyloxy-, Propargyloxy-, Cyanmethyloxy-, Methoxycarbonylmethyloxy-, Cyan-, Halogen-, Nitro-, Amino-, Dialkylamino-, Alkylmercapto-, Alkylsulfinyl-, Alkylsulfonyl-, Alkylcarbonyloxy- oder eine 1-Imidazolylgruppe, wobei die beiden Substituenten gleich oder verschieden sein können und in 2,3-, 2,4-, 2,5-, 2,6-, 3,4- und 3,5-Stellung, bevorzugt jedoch in 2,4-, 2,5- und 3,4-Stellung stehen können und die vorgenannten Alkylreste, allein oder in Kombination mit anderen Resten, 1-3 C-Atome aufweisen können.

Bevorzugte trisubstituierte Phenyle enthalten Hydroxy- und Methoxygruppen als Substituenten.

Bedeutet $R^3$ einen heterocyclischen Fünfring mit 1-4 Heteroatomen, wobei die Heteroatome gleich oder verschieden sein können und Sauerstoff, Stickstoff oder Schwefel bedeuten und gegebenenfalls an einem oder mehreren Stickstoffatomen ein Sauerstoffatom tragen kann, so sind in diesem Sinne bevorzugt der Pyrrol-, Furan-, Thiophen-, Pyrazol-, Imidazol-, Thiazol-, Isothiazol-, Oxazol-, Isoxazol-, Triazol-, Tetrazol-, Thiadiazol-, Oxadiazolrest.

Bedeutet $R^3$ einen heterocyclischen Sechsring, so sind der Pyridin-, N-Oxy-pyridin-, Pyrimidin-, N,N′-Dioxy-pyrimidin, Pyrazin-, N,N′-Dioxy-pyrazin, Pyridazin-, Oxazin-, Thiazin-, Triazin-, Tetrazin- und Chinolinrest bevorzugt.

Alkyl-, Alkoxy- und Alkylmercapto-Substituenten in den heterocyclischen Fünf- und Sechsringen können 1-6, vorzugsweise 1-4 Kohlenstoffatome enthalten. Bevorzugt ist der Methyl-, Ethyl-, Methoxy-, Ethoxy-, Methylmercapto- und Ethylmercaptorest. Unter Halogen ist allgemein Fluor, Chlor und Brom, vorzugsweise Chlor zu verstehen.

Bedeutet X eine Bindung und $R^3$ einen Alkyl-, Alkenyl-, Alkinyl- oder Alkandienylrest, so sind darunter gerade oder verzweigte Ketten mit 2 - 10 vorzugsweise 2 - 6 C-Atomen zu verstehen, die gegebenenfalls durch Halogen substituiert sein können. Bevorzugt in diesem Sinne ist der Ethyl-, Propyl-, Butyl-, Pentyl-, Hexyl-, Vinyl-, Propenyl-, Hexadienyl- und Propinylrest. Bedeutet X eine Bindung und $R^3$ einen Cycloalkyl- oder Cycloalkenylrest, so sind darunter Ringe mit drei bis sieben Gliedern zu verstehen. Bevorzugt in diesem Sinne ist der Cyclopropyl-, Cyclobutyl-, Cyclopentyl-, Cyclohexyl-, Cyclopentenyl- und Cyclohexenylrest. Bedeutet X eine Bindung und $R^3$ einen Alkoxyalkyl-, Alkoxyalkenyl-, Carboxyalkyl-, Alkoxycarbonylalkyl-, Hydroxyalkylrest, so können die Alkyl- oder Alkoxygruppe 1 bis 6 C-Atome enthalten. Bevorzugt in diesem Sinne ist der Ethoxymethyl-, Methoxyethyl-, Ethoxyethyl-, Carboxymethyl-, Carboxypropyl-, Carboxybutyl-, Methoxycarbonylmethyl-, Methoxycarbonylethyl-, Methoxycarbonylpropyl-, Ethoxycarbonylmethyl-, Ethoxycarbonylethyl-, Ethoxycarbonylpropyl-, Propoxycarbonylethyl-, Hydroxymethyl-, Hydroxyethyl-, Hydroxypropyl-, Hydroxybutylrest.

Für X sind in den Gruppen $R^3$-X-CO-NH-, $R^3$-X-NH-CO-NH- und $R^3$-X-O-CO-NH- der Formel I ein Valenzstrich, eine Methylen-, Ethylen-, Propylen-, Butylen- oder die Vinylengruppe bevorzugt. Der Substituent $R^3$-X-A- steht vorzugsweise in 5- oder 6-Stellung des 2,3-Dihydroindolin-2-ons bzw. in 6- oder 7-Stellung

des 1,2,3,4-Tetrahydrochinolin-2-ons.

Bedeutet $R^1$ eine Alkyl-, Alkenyl- oder Cycloalkylgruppe und $R^2$ eine Alkyl-, Alkenyl- oder eine durch eine Alkyl-, Alkoxy-, Alkylamino- oder Dialkylaminogruppe substituierte Carbonylgruppe, so kann jeder der vorgenannten Alkyl- oder Alkenylteile geradkettig oder verzweigt sein und 1-6 bzw. 2-6 Kohlenstoffatome und der genannte Cycloalkylteil 3-7 Kohlenstoffatome enthalten.

Bevorzugt in diesem Sinne ist für $R^1$ ein Wasserstoffatom, die Methyl-, Ethyl-, Isopropyl-, 3-Pentyl-, Cyclopentyl- oder Cyclohexylgruppe. $R^2$ kann vorzugsweise eine Methyl-, Ethyl-, Isopropyl-, 3-Pentyl-, Cyan-, Carboxy-, Acetyl-, Propinyl-, Methoxycarbonyl-, Ethoxycarbonyl-, Aminocarbonyl-, Methylaminocarbonyl-, Dimethylaminocarbonyl- und Hydrazinocarbonylgruppe darstellen.

Bilden $R^1$ und $R^2$ zusammen mit dem C-Atom, an das sie gebunden sind, einen Cycloalkylring, so handelt es sich dabei vorzugsweise um die Spirocyclopropyl-, Spirocyclobutyl-, Spirocyclopentyl- und Spirocyclohexylgruppe. Bilden $R^1$ und $R^2$ zusammen eine Alkyliden- oder Cycloalkylidengruppe, so ist dabei die Isopropyliden- oder Cyclohexylidengruppe bevorzugt.

Wenn R einen Alkylrest mit 1-6 Kohlenstoffatomen darstellt, handelt es sich vorzugsweise um den Methyl-, Ethyl-, Propyl-, Isopropyl-, Isobutyl- oder Cyclopentylrest.

Besonders bevorzugt sind Verbindungen der allgemeinen Formel I, in der $R^3$ den Phenylrest der allgemeinen Formel II bedeutet, in dem

$R^4$ Wasserstoff, die Methansulfonyloxy-, Trifluormethansulfonyloxy-, Methansulfonylamino-, Trifluormethansulfonyl amino-, Methansulfonyl-methylamino-, Trifluormethansulfonylmethylamino-, Methylsulfinylmethyl-, Methylsulfonylmethyl-, Aminocarbonyl-, Aminosulfonyl-, Methylaminosulfonyl-, Dimethylaminosulfonyl-, Acetylamino-, Methylmercapto-, Methylsulfinyl-, Methylsulfonyl-, Hydroxy-, Allyoxy-, Methyl-, Methoxy-, Propargyloxy-, Cyanmethyloxy-, Methoxycarbonylmethyloxy-, Cyan-, Chlor-, Nitro-, Amino-, Dimethylamino-, Trifluormethyl- oder die 1-Imidazolylgruppe,

$R^5$ Wasserstoff, die Methyl-, Methoxy-, Hydroxy-, Dimethylaminogruppe oder Chlor bedeutet,

$R^6$ Wasserstoff oder die Methoxygruppe ist

oder

$R^3$ den Chinolin-, Methylendioxyphenyl-, Furan-, Thiophen-, Pyridin-, Imidazol-, Thiadiazol- oder Pyridazinrest darstellt sowie deren Methyl-, Ethyl-, Methoxy-, Ethoxy-, Methylmercapto-, Ethylmercapto- und chlorsubstituierten Derivate,

oder

$R^3$ für den Fall, daß X eine Bindung darstellt, neben den genannten Gruppen auch die Butyl-, Pentyl-, Hexyl-, Propenyl-, Hexadienyl-, Cyclopentenyl-, Cyclohexyl-, Ethoxyvinylgruppe bedeutet,

und

R ein Wasserstoffatom, eine Methyl-, Ethyl-, Propyl- oder Isopropylgruppe darstellt,

X eine Bindung, die Ethylen-, Propylen- oder Vinylengruppe bedeutet,

$R^1$ ein Wasserstoffatom, oder die Methylgruppe darstellt und

$R^2$ die Methyl-, Ethyl- oder Isopropylgruppe bedeutet, oder $R^1$ und $R^2$ zusammen mit dem C-Atom, an das sie gebunden sind, einen Spirocyclopentylring darstellen, wobei der Substituent in 6-Stellung mit dem 2,3-Dihydroindolin-2-on bzw. in 7-Stellung mit dem 1,2,3,4-Tetrahydrochinolin-2-on verknüpft ist.

Zur Herstellung von Arzneimitteln werden die Substanzen der allgemeinen Formel I in an sich bekannter Weise mit geeigneten pharmazeutischen Trägersubstanzen, Aroma-, Geschmacks-und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formel I und ihre Salze können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungs-mittel, Lösungsvermittler oder Puffer enthält.

Derartige Zusätze sind z.B. Tartrat- und Citratpuffer, Ethanol, Komplexbildner (wie Ethylendiamintetraessigsäure und deren nicht toxische Salze) und hochmolekulare Polymere (wie flüssiges Polyethylenoxid) zur Viskositätsregulierung. Feste Trägerstoffe sind z.B. Stärke, Lactose, Mannit, Methylcellulose, Talkum, hochdisperse Kieselsäuren, hochmolekulare Fettsäuren (wie Staerinsäure), Gelatine, Agar-Agar, Calciumphosphat, Magnesiumstearat, tierische und pflanzliche Fette und feste hochmolekulare Polymere (wie Polyethylenglykole). Für orale Applikation geeignete Zubereitungen können gewünschtenfalls Geschmacks- und Süßstoffe enthalten.

Die Verbindungen werden üblicherweise in Mengen von 10-1500 mg pro Tag bezogen auf 75 kg Körpergewicht appliziert. Bevorzugt ist es, 2-3 mal pro Tag 1-2 Tabletten mit einem Wirkstoffgehalt von 5-500 mg zu verabreichen. Die Tabletten können auch retardiert sein, wodurch nur noch 1 mal pro Tag 1-2 Tabletten mit 20-700 mg Wirkstoff gegeben werden müssen. Der Wirkstoff kann auch durch Injektion 1-8

mal pro Tag bzw. durch Dauerinfusion gegeben werden, wobei Mengen von 10-1000 mg pro Tag normalerweise ausreichen.

Zur Überführung der Verbindungen der allgemeinen Formel I bzw. deren tautomeren Formen in ihre pharmakologisch unbedenklichen Salze, setzt man diese vorzugsweise in einem organischen Lösungsmittel mit der äquivalenten Menge einer anorganischen oder organischen Säure, z.B. Salzsäure, Bromwasserstoffsäure, Phosphorsäure, Schwefelsäure, Essigsäure, Citronensäure, Weinsäure, Maleinsäure, Fumarsäure, Benzoesäure oder Cyclohexylsulfaminsäure um.

Die nachträgliche Umwandlung einer Verbindung der Formel I in eine andere Verbindung der Formel I betrifft z.B. die Oxidation der stickstoffhaltigen Ringe in die entsprechenden N-Oxide, was vorwiegend durch $H_2O_2$ in Essigsäure geschieht, sowie die Hydrierung eines ungesättigten Substituenten. Dies trifft insbesondere für die Hydrierung einer Vinyl-Verbindung (X = -CH=CH-) in die entsprechende Ethyl-Verbindung zu.

Außerdem betrifft die nachträgliche Umwandlung Verbindungen der allgemeinen Formel I in denen $R^1$ oder $R^2$ eine Carboxylgruppe oder ein reaktionsfähiges Derivat wie z.B. Carbonsäureester oder Säurechlorid darstellt, die sich mit Hydrazin, Ammoniak, einem primären oder sekundären Amin oder einem reaktionsfähigen Derivat hiervon zu neuen Verbindungen der allgemeinen Formel I, in denen $R^1$ oder $R^2$ eine durch eine Amino-, Alkylamino-, Dialkylamino- oder Hydrazinogruppe substituierte Carbonylgruppe ist, umsetzen lassen. Die nachträgliche Umwandlung betrifft auch Verbindungen der allgemeinen Formel I in denen $R^1$ oder $R^2$ eine Aminocarbonylgruppe darstellt, zu solchen, in denen $R^1$ oder $R^2$ eine Cyangruppe ist, sowie die nachträgliche Umwandlung einer Cyanogruppe in eine Carboxyl-, Aminocarbonyl- oder Alkoxycarbonylgruppe. Diese Umwandlungen werden alle nach allgemein üblichen und literaturbekannten Methoden vorgenommen.

Die nachträgliche Umwandlung einer Verbindung der allgemeinen Formel I, in der $R^3$ für einen durch Halogen substituierten Alkyl- oder Arylrest steht, in eine andere Verbindung der allgemeinen Formel I betrifft z.B. auch die Überführung der Halogenverbindungen in die entsprechenden Hydroxyverbindungen unter Phasen-Transfer-Bedingungen oder die Umsetzung entsprechender Halogenverbindungen mit einem offenkettigen oder cyclischen sekundären Amin in einem interten Lösungsmittel bei Siedetemperatur des Reaktionsgemisches.

Weiterhin lassen sich durch Abspaltung von Schutzgruppen Verbindungen der allgemeinen Formel I in andere Verbindungen der Formel I überführen.

Verbindungen der allgemeinen Formel I werden nach an sich bekannten Verfahren hergestellt, indem man 5-Amino-2,3-dihydroindolin-2-on-Derivate der allgemeinen Formel III

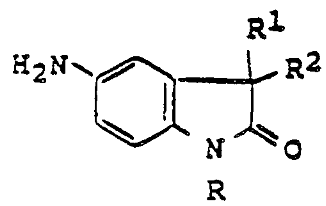

(III)

oder

6-Amino-2,3-dihydroindolin-2-on-Derivate der allgemeinen Formel IV

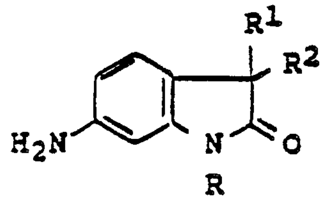

(IV),

oder

6-Amino-1,2,3,4-tetrahydrochinolin-2-on-Derivate der allgemeinen Formel V

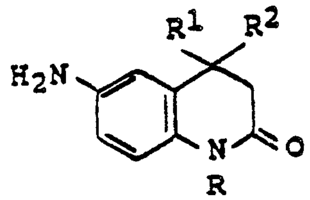

(V),

oder

7-Amino-1,2,3,4-tetrahydrochinolin-2-on-Derivate der allgemeinen Formel VI

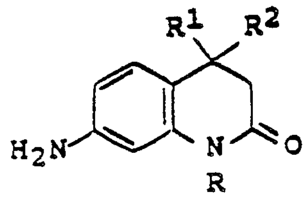

(VI),

in denen R, $R^1$ und $R^2$ die oben angegebene Bedeutung haben,

a) mit einer Verbindung der allgemeinen Formel VII

$R^3$-X-CO-Y (VII),

in der $R^3$ und X die oben angegebene Bedeutung haben und Y einen leicht abspaltbaren Rest, wie ein Halogenatom, eine Methoxy- oder Ethoxygruppe bzw. die Verbindung $R^3$-X-CO-Y ein Anhydrid oder ein anderes aktiviertes Carbonsäurederivat darstellt, umsetzt,

oder

b) mit einem Isocyanat der Formel VIII

$R^3$-X-N=C=O (VIII),

in der $R^3$ und X die oben angegebene Bedeutung haben, umsetzt,

oder

c) mit einer Verbindung der Formel IX

$R^3$-X-O-CO-Y (IX),

in der $R^3$, X und Y die oben angegebene Bedeutung haben, umsetzt

und eine erfindungsgemäß erhaltene Verbindung der allgemeinen Formel I gewünschtenfalls in ein anderes Derivat der Formel I oder dessen Tautomer umwandelt und/oder eine erhaltene Verbindung der allgemeinen Formel I, dessen Tautomer, Enantiomer bzw. Stereoisomer in ein physiologisch verträgliches Salz überführt.

Die 5-Amino- und 6-Amino-2,3-dihydroindolin-2-on-Derivate der Formel III und IV mit R=H wurden analog zur Vorschrift in EP 161 632 A2, EP-A-186,010 und EP-A-189,103 hergestellt.

Die Ausgangsstoffe der allgemeinen Formel III und IV, bei denen R für einen Alkylrest steht, wurden analog zur Vorschrift in der EP-A-268,178 dargestellt.

Die 6-Amino-1,2,3,4-tetrahydrochinolin-2-on-Derivate der allgemeinen Formel V werden nach an sich bekannten Verfahren hergestellt, in dem man Anilin mit einer Verbindung der allgemeinen Formel X

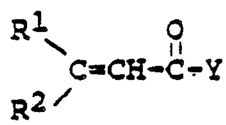

(X),

in der $R^1$, $R^2$ und Y die oben angegebene Bedeutung haben, in einem inerten Lösungsmittel zu einer Verbindung der Formel XI

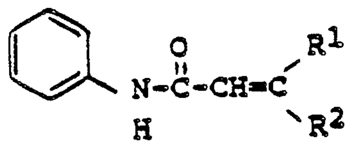

(XI),

umsetzt, unter üblichen Bedingungen zu Verbindungen der allgemeinen Formel XII

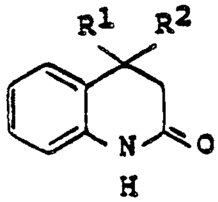

(XII),

in der $R^1$ und $R^2$ die bereits umschriebene Bedeutung haben, cyclisiert, in bekannter Weise durch Nitrierung in Verbindungen der allgemeinen Formel XIII

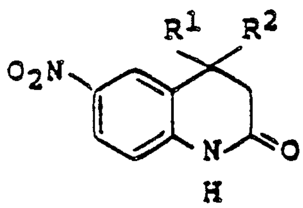

(XIII),

überführt und nach gewünschter N-Alkylierung unter bekannten Bedingungen katalytisch die Nitrogruppe zur Aminogruppe reduziert.

In analoger Weise werden aus 3-Acetamidoanilin durch Umsetzung mit einer Verbindung der allgemeinen Formel X und Cyclisierung Verbindungen der allgemeinen Formel XIV

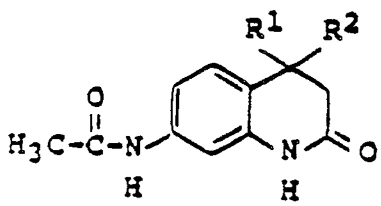

(XIV),

hergestellt, die nach gewünschter N-Alkylierung und durch Abspaltung der Schutzgruppe in die 7-Amino-1,2,3,4-tetrahydrochinolin-2-on-Derivate der allgemeinen Formel VI überführt werden können.

Die Umsetzung der aktivierten Carbonsäurederivate der allgemeinen Formel VII mit Aminen der allgemeinen Formel III, IV, V oder VI zu den entsprechenden Amiden der allgemeinen Formel I findet in inerten Lösungsmitteln, vorzugsweise Dichlormethan, Chloroform oder Dichlorethan, in Gegenwart einer organischen Base, wie Trialkylamin, Pyridin, Dialkylaminopyridin, N-Alkylmorpholin, vorzugsweise Triethylamin oder Pyridin, statt. Flüssige organische Basen können dabei gegebenenfalls als Solvens genutzt werden.

Die Reaktion der Isocyanate der Formel VIII mit Verbindungen der allgemeinen Formel III, IV, V oder VI zu den entsprechenden Harnstoffderivaten der allgemeinen Formel I wird ebenfalls in inerten Lösungsmitteln wie Tetrahydrofuran, Dioxan, Benzol, Chloroform, Dichlormethan, vorzugsweise Dioxan, durchgeführt.

Die Urethane der allgemeinen Formel I, bei denen A in dem Substituenten $R^3$-X-A- für die -O-CO-NH-Gruppe steht, lassen sich vorzugsweise durch Umsetzung der Chlorameisensäureester der Formel IX, in der Y gleich Chlor ist, mit Verbindungen der allgemeinen Formel III, IV, V oder VI in Dichlormethan oder einem anderen der oben erwähnten inerten Lösungsmittel herstellen.

Die Reaktionen in den oben genannten Verfahren a) - c) zu den gewünschten Verbindungen der allgemeinen Formel I werden in den beschriebenen Lösungsmitteln bei Temperaturen zwischen - 10 °C und

Siedetemperatur des Reaktionsgemisches, vorzugsweise jedoch bei Raumtemperatur, durchgeführt.

Die Ausgangsstoffe für die beanspruchten 1,2,3,4-Tetrahydrochinolin-2-on-Derivate der allgemeinen Formel I können wie folgt dargestellt werden:

a) N-Acetyl-3-(3,3-dimethylacryloylamino)anilin

56.7 g (0.42 mol) 3-Acetamidoanilin in 400 ml Dichlormethan und 65 ml (0.46 ml) Triethylamin werden unter Eiskühlung mit 59.0 g (0.46 mol) 3,3-Dimethylacryloylchlorid versetzt und 2 h bei Raumtemperatur gerührt. Die Lösung wird danach mit Wasser extrahiert, die abgetrennte organische Phase über $Na_2SO_4$ getrocknet, eingeengt und mit Essigester versetzt. Beim Stehen im Kühlschrank scheiden sich Kristalle ab, Ausb. 45.7 g (47 % d. Th.), Schmp. 144-148° C.

b) 7-Acetamido-1,2,3,4-tetrahydro-4,4-dimethylchinolin-2-on

23.2 g (0.1 mol) N-Acetyl-3-(3,3-dimethylacryloylamino)anilin, 92.8 g $AlCl_3$ und 60 g NaCl werden gut durchmischt für 2 h auf 100° C erhitzt. Danach versetzt man mit Eis, rührt kurz nach und saugt das Kristallisat ab. Ausb. 21.4 g (92 % d.Th.) Schmp. 273-278° C.

c) 7-Amino-1,2,3,4-tetrahydro-4,4-dimethylchinolin-2-on

Eine Suspension aus 23.2 g (0.1 mol) 7-Acetamido-1,2,3,4-tetrahydro-4,4-dimethylchinolin-2-on, 200 ml Ethanol und 20 ml konz. Salzsäure wird bis zum vollständigen Lösen unter Rückfluß gekocht. Dann wird mit Ammoniak auf pH 8 eingestellt, 3 mal mit Dichlormethan extrahiert, die organische Phase über $Na_2SO_4$ getrocknet und vom Lösungsmittel befreit. Man erhält 13.1 g (69 % d.Th.), Schmp. 161-163° C.

d) N-Phenyl-3,3-dimethylacrylsäureamid

wird analog zu a) aus Anilin und 3,3-Dimethylacryloylchlorid hergestellt. Ausb. 86 % d.Th., Schmp. 124-126° C.

e) 1,2,3,4-Tetrahydro-4,4-dimethylchinolin-2-on

Die Cyclisierung wird analog zu Verfahren b) durchgeführt. Ausb. 68 % d.Th. Schmp. 113-115° C.

f) 1,2,3,4-Tetrahydro-4,4-dimethyl-6-nitrochinolin-2-on

Zu 17.5 g (0.1 mol) 1,2,3,4-Tetrahydro-4,4-dimethylchinolin-2-on in 200 ml 80proz. Schwefelsäure tropft man unter Eiskühlung eine Mischung aus 5 ml 96proz. Salpetersäure und 20 ml 80proz. Schwefelsäure und rührt eine Stunde bei Raumtemperatur. Anschließend gießt man das Reaktionsgemisch auf Eis, saugt das Kristallisat ab und wäscht mit Wasser nach. Das Rohprodukt wird durch Säulenchromatographie an Kieselgel mit 2-Butanon/Essigester 20/1 als Eluens gereinigt. Ausb. 17.2 g (78 % d.Th.), Schmp. 202-204° C.

g) 6-Amino-1,2,3,4-tetrahydro-4,4-dimethylchinolin-2-on

22 g (0.1 mol) 1,2,3,4-Tetrahydro-4,4-dimethyl-6-nitrochinolin-2-on in 500 ml Methanol werden nach Zugabe von 2 g Palladium auf Kohle (10 %) bei Atmosphärendruck hydriert. Nach Abtrennen des Katalysators wird das Lösungsmittel im Vakuum entfernt. Ausb. 18.4 g (97 % d.Th.), Schmp. 126-130° C.

Bevorzugt im Sinne der vorliegenden Erfindung sind außer den in den Beispielen genannten Verbindungen und den durch Kombination aller in den Ansprüchen genannten Bedeutungen ableitbaren Verbindungen die folgenden Amide, Harnstoffe und Urethane sowie deren Tautomeren.

6-(3,3-Dimethylhexanoylamino)-2,3-dihydro-3,3-dimethyl-(1H)-indolin-2-on
6-(6-Methylheptanoylamino)-2,3-dihydro-3,3-dimethyl-(1H)-indolin-2-on
6-(2-Chlor-2-methylpropanoylamino)-2,3-dihydro-3,3-dimethyl-(1H)-indolin-2-on
6-(6-Aminohexanoylamino)-2,3-dihydro-3,3-dimethyl-(1H)-indolin-2-on
6-[(3-Methylpyrazol-5-yl)carbonylamino]-2,3-dihydro-3,3-dimethyl- (1H)-indolin-2-on
6-[(3-Phenylpyrazol-5-yl)carbonylamino]-2,3-dihydro-3,3-dimethyl-(1H)-indolin-2-on
6-[(1,2,5-Thiadiazol-3-yl)carbonylamino]-2,3-dihydro-3,3-dimethyl-(1H)-indolin-2-on
6-[(3-Methylpyrazol-5-yl)acryloylamino]-2,3-dihydro-3,3-dimethyl-(1H)-indolin-2-on
6-(4-Allyloxybenzoylamino)-2,3-dihydro-3,3-dimethyl-(1H)-indolin-2-on
6-(3-Hydroxy-2,4-dimethoxybenzoylamino)-2,3-dihydro-3,3-dimethyl-(1H)-indolin-2-on
6′-Heptanoylamino-1′,2′-dihydrospiro[cyclopentan-1,3′-(3H)-indolin-2′-on]
6′-[(4-Methoxyphenyl)acetamido]-1′,2′-dihydrospiro[cyclopentan-1,3′-(3H)-indolin-2′-on]
6′-[(4-(4-Methoxyphenyl)butanoylamino]-1′,2′-dihydrospiro[cyclopentan-1,3′-(3H)-indolin-2′-on]
6′-[5-(4-Methoxyphenyl)pentanoylamino]-1′,2′-dihydrospiro[cyclopentan-1,3′-(3H)-indolin-2′-on]
7-Benzoylamino-1,2,3,4-tetrahydro-4,4-dimethylchinolin-2-on
7-Heptanoylamino-1,2,3,4-tetrahydro-4,4-dimethylchinolin-2-on
7-Methoxyacetamido-1,2,3,4-tetrahydro-4,4-dimethylchinolin-2-on
7-(3-Ethoxyacryloylamino)-1,2,3,4-tetrahydro-4,4-dimethylchinolin-2-on
7-(3-Ethoxypropionylamino)-1,2,3,4-tetrahydro-4,4-dimethylchinolin-2-on
7-Pivaloylamino-1,2,3,4-tetrahydro-4,4-dimethylchinolin-2-on
7-(6-Bromhexanoylamino)-1,2,3,4-tetrahydro-4,4-dimethylchinolin-2-on
7-(6-Hydroxyhexanoylamino)-1,2,3,4-tetrahydro-4,4-dimethylchinolin-2-on
7-Cinnanoylamino-1,2,3,4-tetrahydro-4,4-dimethylchinolin-2-on
7-(2-Methoxycinnamoylamino)-1,2,3,4-tetrahydro-4,4-dimethylchinolin-2-on
7-(3-Methoxycinnamoylamino)-1,2,3,4-tetrahydro-4,4-dimethylchinolin-2-on
7-(4-Methoxycinnamoylamino)-1,2,3,4-tetrahydro-4,4-dimethylchinolin-2-on
7-(4-Trifluormethylcinnamoylamino)-1,2,3,4-tetrahydro-4,4-dimethylchinolin-2-on
7-(4-Hydroxycinnamoylamino)-1,2,3,4-tetrahydro-4,4-dimethylchinolin-2-on
7-(4-Cyanocinnamoylamino)-1,2,3,4-tetrahydro-4,4-dimethylchinolin-2-on
7-(4-Pyridylacryloylamino)-1,2,3,4-tetrahydro-4,4-dimethylchinolin-2-on
7-(3-Pyridylacryloylamino)-1,2,3,4-tetrahydro-4,4-dimethylchinolin-2-on
7-(4-Methylsulfonylcinnamoylamino)-1,2,3,4-tetrahydro-4,4-dimethylchinolin-2-on
7-(2-Hydroxybenzoylamino)-1,2,3,4-tetrahydro-4,4-dimethylchinolin-2-on
7-(2-Acetoxybenzoylamino)-1,2,3,4-tetrahydro-4,4-dimethylchinolin-2-on
7-(2-Methoxybenzoylamino)-1,2,3,4-tetrahydro-4,4-dimethylchinolin-2-on
7-(3-Hydroxybenzoylamino)-1,2,3,4-tetrahydro-4,4-dimethylchinolin-2-on
7-(4-Fluorbenzoylamino)-1,2,3,4-tetrahydro-4,4-dimethylchinolin-2-on
7-(4-Hydroxybenzoylamino)-1,2,3,4-tetrahydro-4,4-dimethylchinolin-2-on
7-(4-Methylbenzoylamino)-1,2,3,4-tetrahydro-4,4-dimethylchinolin-2-on
7-(4-Trifluormethylbenzoylamino)-1,2,3,4-tetrahydro-4,4-dimethylchinolin-2-on
7-[4-(Imidazol-1-yl)benzoylamino]-1,2,3,4-tetrahydro-4,4-dimethylchinolin-2-on
7-(4-Methylsulfonyl-2-methoxy-benzoylamino)-1,2,3,4-tetrahydro-4,4-dimethylchinolin-2-on
7-(4-tert.-Butylbenzoylamino)-1,2,3,4-tetrahydro-4,4-dimethylchinolin-2-on
7-(4-Cyanbenzoylamino)-1,2,3,4-tetrahydro-4,4-dimethylchinolin-2-on
7-(4-Methylsulfonyl)benzoylamino -1,2,3,4-tetrahydro-4,4-dimethylchinolin-2-on
7-Isonicotinoylamino-1,2,3,4-tetrahydro-4,4-dimethylchinolin-2-on
7-(2-Methylisonicotinoylamino)-1,2,3,4-tetrahydro-4,4-dimethylchinolin-2-on
7-(Chinolin-4-yl)carbonylamino-1,2,3,4-tetrahydro-4,4-dimethylchinolin-2-on
7-(4-Allyloxybenzoylamino)-1,2,3,4-tetrahydro-4,4-dimethylchinolin-2-on
7-(3-Allyloxybenzoylamino)-1,2,3,4-tetrahydro-4,4-dimethylchinolin-2-on
7-(3-Hydroxy-2,4-dimethoxybenzoylamino)-1,2,3,4-tetrahydro-4,4-dimethylchinolin-2-on
N-(4-Methoxybenzyl-N′-[2,3-dihydro-3,3-dimethyl-2-oxo-(1H)indol-6-yl]harnstoff
N-(4-Hydroxyphenyl)-N′-[2,3-dihydro-3,3-dimethyl-2-oxo-(1H)indol-6-yl]harnstoff
N-[2-(4-Hydroxyphenyl)ethyl]-N′-[2,3-dihydro-3,3-dimethyl2-oxo-(1H)indol-6-yl]harnstoff
N-[2-(2,4-Dimethoxyphenyl)ethyl]-N′-[2,3-dihydro-3,3-dimethyl-2-oxo-(1H)indol-6-yl]harnstoff
N-(4-Allyloxyphenyl)-N′-[2,3-dihydro-3,3-dimethyl-2-oxo-(1H)indol-6-yl]harnstoff
N-(4-Trifluormethylphenyl)-N′-[2,3-dihydro-3,3-dimethyl-2-oxo-(1H)indol-6-yl]harnstoff
N-(2-Phenylethyl)-N′-[1,2,3,4-tetrahydro-4,4-dimethyl-2-oxochinolin-7-yl]harnstoff

N-(3-Phenylpropyl)-N′-[1,2,3,4-tetrahydro-4,4-dimethyl-2-oxochinolin-7-yl]harnstoff
N-(2-Methoxyphenyl)-N′-[1,2,3,4-tetrahydro-4,4-dimethyl-2-oxochinolin-7-yl]harnstoff
N-(3-Trifluormethylphenyl)-N′-[1,2,3,4-tetrahydro-4,4-dimethyl-2-oxochinolin-7-yl]harnstoff
N-[3,4-Dimethoxyphenyl)-N′-[1,2,3,4-tetrahydro-4,4-dimethyl-2-oxochinolin-7-yl]harnstoff
N-(4-Cyanphenyl)-N′-[1,2,3,4-tetrahydro-4,4-dimethyl-2-oxochinolin-7-yl]harnstoff
N-[2-(4-Methoxyphenyl)ethyl)-N′-[1,2,3,4-tetrahydro-4,4-dimethyl-2-oxochinolin-7-yl]harnstoff
N-(4-Allyloxyphenyl)-N′-[1,2,3,4-tetrahydro-4,4-dimethyl-2-oxochinolin-7-yl]harnstoff
N-(2-Phenylethyl)-N′-[1′,2′-dihydro-2′-oxospiro(cyclopentan-1,3′-(3H)indol)-6′-yl]harnstoff
N-(3-Phenylpropyl)-N′-[1′,2′-dihydro-2′-oxospiro(cyclopentan-1,3′-(3H)indol)-6′-yl]harnstoff
N-(2-Methoxyphenyl)-N′-[1′,2′-dihydro-2′-oxospiro(cyclopentan-1,3′-(3H)indol)-6′-yl]harnstoff
N-(3,4-Dimethoxyphenyl)-N′-[1′,2′-dihydro-2′-oxospiro(cyclopentan-1,3′-(3H)indol)-6′-yl]harnstoff
N-(4-Cyanphenyl)-N′-[1′,2′-dihydro-2′-oxospiro(cyclopentan-1,3′-(3H)indol)-6′-yl]harnstoff
N-(4-Allyloxyphenyl)-N′-[1′,2′-dihydro-2′-oxospiro(cyclopentan-1,3′-(3H)indol)-6′-yl]harnstoff
N-Hexyl-N′-[1′,2′-dihydro-2′-oxospiro(cyclopentan-1,3′-(3H)indol)-6′-yl]harnstoff
N-[2,3-Dihydro-3,3-dimethyl-2-oxo-(1H)indol-6-yl]carbamidsäure-[2-(4-methoxyphenyl)ethyl]ester
N-[2,3-Dihydro-3,3-dimethyl-2-oxo-(1H)indol-6-yl]carbamidsäure-phenylester
N-[2,3-Dihydro-3,3-dimethyl-2-oxo-(1H)indol-6-yl]carbamidsäure-(4-methoxyphenyl)ester
N-[2,3-Dihydro-3,3-dimethyl-2-oxo-(1H)indol-6-yl]carbamidsäure-(4-trifluormethylphenyl)ester
N-[2,3-Dihydro-3,3-dimethyl-2-oxo-(1H)indol-6-yl]carbamidsäure-benzylester
N-[2,3-Dihydro-3,3-dimethyl-2-oxo-(1H)indol-6-yl]carbamidsäure-(3-phenylpropyl)ester
N-[2,3-Dihydro-3,3-dimethyl-2-oxo-(1H)indol-6-yl]carbamidsäure-(2-phenylethyl)ester
N-[1,2,3,4-Tetrahydro-4,4-dimethyl-2-oxochinolin-7-yl]carbamidsäure-hexylester
N-[1,2,3,4-Tetrahydro-4,4-dimethyl-2-oxochinolin-7-yl]carbamidsäure-(2-phenylethylester
N-[1,2,3,4-Tetrahydro-4,4-dimethyl-2-oxochinolin-7-yl]carbamidsäure-[2-(4-methoxyphenyl)ethyl]ester
N-[1,2,3,4-Tetrahydro-4,4-dimethyl-2-oxochinolin-7-yl]carbamidsäure-phenylester
N-[1,2,3,4-Tetrahydro-4,4-dimethyl-2-oxochinolin-7-yl]carbamidsäure-(4-methoxyphenyl)ester
N-[1,2,3,4-Tetrahydro-4,4-dimethyl-2-oxochinolin-7-yl]carbamidsäure-(4-trifluormethylphenyl)ester
N-[1,2,3,4-Tetrahydro-4,4-dimethyl-2-oxochinolin-7-yl]carbamidsäure-(3-phenylpropyl)ester

Die nachfolgenden Beispiele zeigen einige der Verfahrensvarianten, die zur Synthese der erfindungsgemäßen Verbindungen verwendet werden können. Sie sollten jedoch keine Einschränkung des Erfindungsgegenstandes darstellen. Struktur und Reinheit der Verbindungen sind durch NMR-Spektroskopie, Massenspektrometrie und C,H,N-Analyse bestimmt worden.

## Beispiel A

### 6-Benzoylamino-2,3-dihydro-3,3-dimethyl-(1H)-indolin-2-on

Zu einer Suspension von 5.28 g (0.03 mol) 6-Amino-2,3-dihydro-3,3-dimethyl-(1H)-indolin-2-on und 4.6 ml Triethylamin in 50 ml abs. Dichlormethan wird bei 0 °C innerhalb von 15 min 4.36 g (0.031 mol) Benzoylchlorid getropft und eine Stunde bei Raumtemperatur gerührt. Dann wird mit 200 ml Eiswasser versetzt, der Niederschlag abgesaugt, mit Wasser gewaschen und aus Methanol umkristallisiert. Ausbeute 7.8 g (93 % d.Th.), Schmp. 251-254 °C.

In analoger Weise wurden folgende Verbindungen hergestellt:

| | Verbindung | Schmelzpunkt [°C] |
|---|---|---|
| 1 | 6-Heptanoylamino-2,3-dihydro-3,3-dimethyl-(1H)-indolin-2-on | 142-145 |
| 2 | 6-Methoxyacetamido-2,3-dihydro-3,3-dimethyl-(1H)-indolin-2-on | 160-163 |
| 3 | 6-(3-Ethoxyacryloylamino)-2,3-dihydro-3,3-dimethyl-(1H)-indolin-2-on | 240-243 |
| 4 | 6-(3-Ethoxypropionylamino)-2,3-dihydro-3,3-dimethyl-(1H)-indolin-2-on | 138-140 |
| 5 | 6-(6-Bromhexanoylamino)-2,3-dihydro-3,3-dimethyl-(1H)-indolin-2-on | 155-158 |
| 6 | 6-[(2-Methoxyphenyl)acetamido)-2,3-dihydro-3,3-dimethyl-(1H)-indolin-2-on | 188-192 |
| 7 | 6-ʳ(4-Methoxyphenyl)acetamido)-2,.-dihydro-3,3-dimethyl-(1H)-indolin-2-on | 170-174 |
| 8 | 6-[4-(Imidazol-1-yl)benzoylamino]-2,3-dihydro-3,3-dimethyl-(1H)-indolin-2-on | 303-308 |
| 9 | 6-(2-Acetoxybenzoylamino)-2,3-dihydro-3,3-dimethyl-(1H)-indolin-2-on | 190-193 |
| 10 | 6-(2-Methoxybenzoylamino)-2,3-dihydro-3,3-dimethyl-(1H)-indolin-2-on | 190-192 |
| 11 | 6-(4-Fluorbenzoylamino)-2,3-dihydro-3,3-dimethyl-(1H)-indolin-2-on | 241-245 |
| 12 | 6-(4-Methoxybenzoylamino)-2,3-dihydro-3,3-dimethyl-(1H)-indolin-2-on | 253-255 |
| 13 | 6-(4-Methylbenzoylamino)-2,3-dihydro-3,3-dimethyl-(1H)-indolin-2-on | 274-278 |
| 14 | 6-(4-Trifluormethylbenzoyl-amino)-2,3-dihydro-3,3-dimethyl-(1H)-indolin-2-on | 244-246 |

| | Verbindung | Schmelzpunkt [°C] |
|---|---|---|
| 15 | 6-[3-(Imidazol-1-yl)benzoylamino]-2,3-dihydro-3,3-dimethyl-(1H)-indolin-2-on | 291-297 |
| 16 | 6-(4-Methylsulfonyl-2-methoxy-benzoylamino)-2,3-dihydro-3,3-dimethyl-(1H)-indolin-2-on | 270-273 |
| 17 | 6-(4-tert.-Butylbenzoylamino)-2,3-dihydro-3,3-dimethyl-(1H)-indolin-2-on | 218-220 |
| 18 | 6-(2-Thienylcarbonylamino)-2,3-dihydro-3,3-dimethyl-(1H)-indolin-2-on | 268-271 |
| 19 | 6-(Chinolin-4-yl)carbonylamino-2,3-dihydro-3,3-dimethyl-(1H)-indolin-2-on | 295-297 |
| 20 | 6-(2-Furoylamino)-2,3-dihydro-3,3-dimethyl-(1H)-indolin-2-on | 271-274 |
| 21 | 6- Imidazol-5-yl)carbonylamino-2,3-dihydro-3,3-dimethyl-(1H)-indolin-2-on | >300 |
| 22 | 6-(1,2,3-Thiadiazol-4-yl)carbonyl-amino-2,3-dihydro-3,3-dimethyl-(1H)-indolin-2-on | 255-258 (Zers.) |
| 23 | 6-(2-Thienyl)acetamido-2,3-dihydro-3,3-dimethyl-(1H)-indolin-2-on | 222-225 |
| 24 | 6-(4-Benzyloxybenzoylamino)-2,3-dihydro-3,3-dimethyl-(1H)-indolin-2-on | 225-228 |
| 25 | 6'-[3-(4-Methoxyphenyl)propionyl-amino]-1',2'-dihydrospiro-[cyclo-pentan-1,3'-(3H)indolin-2'-on] | 222-225 |
| 26 | 6'-Benzoylamino-1',2'-dihydrospiro-[cyclopentan-1,3'-(3H)indolin-2'-on] | 223-226 |
| 27 | 6'-(4-Benzyloxybenzoylamino)-1',2'-dihydrospiro-[cyclopentan-1,3'-(3H)indolin-2'-on] | 200-203 |

| | Verbindung | Schmelzpunkt [°C] |
|---|---|---|
| 28 | 6'-(4-Methoxybenzoylamino)-1',2'-dihydrospiro-[cyclopentan-1,3'-(3H)indolin-2'-on] | 251-253 |
| 29 | 6'-(4-Methylbenzoylamino)-1',2'-dihydrospiro-[cyclopentan-1,3'-(3H)indolin-2'-on] | 256-259 |
| 30 | 6'-(4-tert.-Butylbenzoylamino)-1',2'-dihydrospiro-[cyclopentan-1,3'-(3H)indolin-2'-on] | 243-245 |
| 31 | 6'-(2-Furoylamino)- 1',2'-dihydrospiro-[cyclopentan-1,3'-(3H)indolin-2'-on] | 254-255 |
| 32 | 6'-(2-Thienylcarbonylamino)-1',2'-dihydrospiro-[cyclopentan-1,3'-(3H)indolin-2'-on] | 272-274 |
| 33 | 6'-(Pyridazin-4-yl)carbonylamino-1' 2'-dihydrospiro-[cyclopentan-1,. '-(3H)indolin-2'-on] | 239-241 |
| 34 | 6'-(4-Trifluormethylbenzoylamino)-1',2'-dihydrospiro-[cyclopentan-1,3'-(3H)indolin-2'-on] | 238-241 |
| 35 | 6'-(4-Cyanbenzoylamino)-1',2'-dihydrospiro-[cyclopentan-1,3'-(3H)indolin-2'-on] | 283-289 |
| 36 | 6-Benzoyl-2,3-dihydro-3-isopropyliden-(1H)-indolin-2-on | 282-283 |
| 37 | 6-Benzoylamino-2,3-dihydro-3-cyclopentyliden-(1H)-indolin-2-on | 279-281 |
| 38 | 5-(3-Ethoxyacryloylamino)-2,3-dihydro-3,3-dimethyl-(1H)-indolin-2-on | 196-198 |
| 39 | 5-Benzoylamino-2,3-dihydro-3,3-dimethyl-(1H)-indolin-2-on | 210-212 |
| 40 | 5-(2-Methoxybenzoylamino)-2,3-dihydro-3,3-dimethyl-(1H)-indolin-2-on | 186-188 |
| 41 | 5-(2-Furoylamino)-2,3-dihydro-3,3-dimethyl-(1H)-indolin-2-on | 223-225 |

| | Verbindung | Schmelzpunkt [°C] |
|---|---|---|
| 42 | 5-(2-Thienylcarbonylamino)-2,3-dihydro-3,3-dimethyl-(1H)-indolin-2-on | 245-246 |
| 43 | 7-Isonicotinoylamino-1,2,3,4-tetrahydro-4,4-dimethylchinolin-2-on | 293-298 |
| 44 | 7-(3-Ethoxyacryloylamino)-1,2,3,4-tetrahydro-4,4-dimethylchinolin-2-on | 208-211 |
| 45 | 7-(4-Methoxybenzoylamino)-1,2,3,4-tetrahydro-4,4-dimethylchinolin-2-on | 269-273 |
| 46 | 6-(2-Benzyloxybenzoylamino)-2,3-dihydro-3,3-dimethyl-(1H)-indolin-2-on | 208-213 |
| 47 | 6-(4-Methoxycinnamoylamino)-2,3-dihydro-3-ethoxycarbonyl-3-rethyl-(1H)-indolin-2-on | 257-258 |
| 48 | 6-Benzoylamino-2,3-dihydro-3-ethoxycarbonyl-3-methyl-(1H)-indolin-2-on | 206-207 |
| 49 | 6-Butanoylamino-2,3-dihydro-3-ethoxycarbonyl-3-methyl-(1H)-indolin-2-on | 82-86 |
| 50 | 6-(2-Chlorpropanoylamino)-2,3-dihydro-3,3-dimethyl-(1H)-indolin-2-on | 234-237 |
| 51 | 6-(3-Trifluormethyl-2-butenoylamino)-2,3-dihydro-3,3-dimethyl-(1H)-indolin-2-on | 232-235 |
| 52 | 6-(3-Benzyloxybenzoylamino)-2,3-dihydro-3,3-dimethyl-(1H)-indolin-2-on | 207-208 |
| 53 | 6-Pivaloylamino-2,3-dihydro-3,3-dimethyl-(1H)-indolin-2-on | 242-245 |
| 54 | 6-(Cyclohexylcarbonylamino)-2,3-dihydro-3,3-dimethyl-(1H)-indolin-2-on | 286-288 |
| 55 | 6-(4-Cyanobenzoylamino)-2,3-dihydro-3,3-dimethyl-(1H)-indolin-2-on | 296-298 |

| | Verbindung | Schmelzpunkt [°C] |
|---|---|---|
| 56 | 6-(4-Methylsulfonylbenzoylamino)-2,3-dihydro-3,3-dimethyl-(1H)-indolin-2-on | 294-298 |
| 57 | 6-(3-Chlorbenzoylamino)-2,3-dihydro-3,3-dimethyl-(1H)-indolin-2-on | 206-211 |
| 58 | 6-(3-Allyloxybenzoylamino)-2,3-dihydro-3,3-dimethyl-(1H)-indolin-2-on | 169-171 |
| 59 | 6-[4-(N,N-Diethylamino)-2-methoxy-benzoylamino]-2,3-dihydro-3,3-dimethyl-(1H)-indolin-2-on | 280-282 |
| 60 | 6'-(3-Ethoxyacryloylamino)-1',2'-dihydrospiro-[cyclopentan-1,3'-(3H)indolin-2'-on] | 198-203 |
| 61 | 6'-(4-Acetamidobenzoylamino)1',2'-dihydrospiro-[cyclopentan-1,3'-(3H)indolin-2'-on] | 264-265 |
| 62 | 6'-(4-Methylsulfonylbenzoylamino)-1',2'-dihydrospiro-[cyclopentan-1,3'-(3H)indolin-2'-on] | 256-259 |
| 63 | 6'-(3-Benzyloxybenzoylamino)-1',2'-dihydrospiro-[cyclopentan-1,3'-(3H)indolin-2'-on] | 199-203 |
| 64 | 5-(4-Cyanbenzoylamino)-2,3-dihydro-3,3-dimethyl-(1H)-indolin-2-on | 280-286 |
| 65 | 6-Benzoylamino-1-ethyl-2,3-dihydro-3,3-dimethyl-indolin-2-on | 172-174 |
| 66 | 6-Benzoylamino-1-(2-propyl)-2,3-dihydro-3,3-dimethylindolin-2-on | 146-148 |

Beispiel B

6-(6-Hydroxyhexanoylamino)-2,3-dihydro-3,3-dimethyl-(1H)-indolin-2-on

3.53 g (0.01 mol) 6-(6-Bromhexanoylamino)-2,3-dihydro-3,3-dimethyl-(1H)-indolin-2-on (Bsp. A, Nr. 5) werden in 30 ml THF gelöst und nach Zugabe von 11 g Amberlyst A 27 in der Carbonat-Form 12 Stunden unter Rückfluß erhitzt. Der Katalysator wird danach abgetrennt, mit Methanol gewaschen und das Filtrat vom Lösungsmittel befreit. Der Rückstand wird durch Flash-Säulenchromatographie mit Dichlormethan/Methanol 18/1 gereinigt. Ausbeute 1.33 g (46 % d.Th.), Schmp. 145-147° C.

Beispiel C

6-(4-Hydroxybenzoylamino)-2,3-dihydro-3,3-dimethyl-(1H)-indolin-2-on

3.86 g (0.01 mol) 6-(4-Benzyloxybenzoylamino)-2,3-dihydro-3,3-dimethyl-(1H)-indolin-2-on (Bsp. A, Nr. 24) werden in 50 ml Methanol unter Zusatz von 0.5 g Palladium auf Kohle (10 %) bei Atmosphärendruck hydriert. Der Katalysator wird abgetrennt, das Filtrat im Vakuum eingedampft und der Rückstand durch Zugabe von Ether zur Kristallisation gebracht. Ausbeute 2.46 g (83 % d. Th.), Schmp. 228-231 °C.

In analoger Weise werden folgende Verbindungen hergestellt.

| | Verbindung | Schmelzpunkt [°C] |
|---|---|---|
| 1 | 6-(2-Hydroxybenzoylamino)-2,3-dihydro-3,3-dimethyl-(1H)-inolin-2-on aus Bsp. A, Nr. 46 | 214-215 |
| 2 | 6-(3-Hydroxybenzoylamino)-2,3-dihydro-3,3-dimethyl-(1H)-indolin-2-on aus Bsp. A, Nr. 52 | 115-117 (Zers.) |
| 3 | 6′-(4-Hydroxybenzoylamino)-1′,2′-dihydrospiro-[cyclopentan-1,3′-(3H)-indolin-2′-on] aus Bsp. A, Nr. 27 | 242-244 |
| 4 | 6′-(3-Hydroxybenzoylamino)-1′,2′,dihydrospiro-[cyclopentan-1,3′-(3H)-indolin-2′-on] aus Bsp. A, Nr. 63 | 126-128 (Zers.) |

Beispiel D

6′(4-Aminobenzoylamino)-1′-2′-dihydrospiro[cyclopentan-1,3′-(3H)-indolin-2′-on]

2,5 g (6.9 mmol) 6′-(4-Acetamidobenzoylamino)-1′-2′-dihydrospiro[cyclopentan-1,3′-(3H)-indolin-2′-on (Bsp. A, Nr. 61) werden in einer Mischung aus 50 ml 6 N Salzsäure und 50 ml Methanol 6 h bei 50oC gerührt. Danach wird die Lösung filtriert, das Filtrat auf ein Viertel des Volumens eingedampft, mit Wasser verdünnt und über Nacht im Kühlschrank stehengelassen. Die cremefarbenen Kristale werden abgesaugt, mit Wasser gewaschen und im Vakuum getrocknet.
Ausbeute 1.77 g (80 % d.Th), Schmp. 221-228 °C.

Beispiel E

6-Cinnamoylamino-2,3-dihydro-3,3-dimethyl-(1H)-indolin-2-on

Eine Lösung aus 3.52 g (0.02 mol) 6-Amino-2,3-dihydro-3,3-dimethyl-(1H)-indolin-2-on in 35 ml abs. Pyridin wird bei 0-10 °C portionsweise mit 3.33 g (0.02 mol) Zimtsäurechlorid versetzt und vier Stunden bei Raumtemperatur gerührt. Danach versetzt man mit 200 ml Eiswasser, säuert mit 6 N HCl an, saugt den Niederschlag ab und wäscht mit Wasser nach. Ausbeute 5.57 g (91 % d.Th.), Schmp. 272-275 °C (MeOH).

In analoger Weise werden folgende Verbindungen hergestellt:

| | Verbindung | Schmelzpunkt [°C] |
|---|---|---|
| 1 | 6-′2-Methoxycinnamoylamino)-2, -dihydro-3,3-dimethyl-(1H)-indolin-2-on | 249- 250 |
| 2 | 6-(4-Methoxycinnamoylamino)-2,3-dihydro-3,3-dimethyl-(1H)-indolin-2-on | 305-308 |
| 3 | 6-[(4-Trifluormethyl)cinnamoylamino]-2,3-dihydro-3,3-dimethyl-(1H)-indolin-2-on | 313-316 |
| 4 | 6-Hexadienoylamino-2,3-dihydro-3,3-dimethyl-(1H)-indolin-2-on | 247-249 |
| 5 | 6′-(4-Methoxycinnamoylamino)-1′,2′-dihydrospiro[cyclopentan-1,3′-(3H)indolin-2′-on] | 297-301 |
| 6 | 6-(4-Cyancinnamoylamino)-2,3-dihydro-3,3-dimethyl-(1H)-indolin 2-on | 315-320 |
| 7 | 6-(4-Methylsulfonylcinnamoylamino)-2,3-dihydro-3,3-dimethyl-(1H)-indolin-2-on | 297-300 |
| 8 | 6-(3-Methoxycinnamoylamino)-2,3-dihydro-3,3-dimethyl-(1H)-indolin-2-on | 215-217 |

Beispiel F

6-(4-Hydroxycinnamoylamino)-2,3-dihydro-3,3-dimethyl-(1H)-indolin-2-on

1.5 g (8.5 mmol) 6-Amino-2,3-dihydro-3,3-dimethyl-(1H)-indolin-2-on und 1.4 g (8.5 mmol) p-Hydroxyzimtsäure in 15 ml abs. THF werden bei RT mit 2 g (9.7 mmol) DCC versetzt und 5 h gerührt. Dann wird der Niederschlag abgesaugt, das Filtrat vom Lösungsmittel befreit und der Rückstand durch Säulenchromatographi an Kieselgel 60 mit Dichlormethan/5proz. Methanol gereinigt. Ausbeute 1.29 g (47 % d.Th.) nach Umkristallisation aus Ethanol/Wasser. Schmp. 243-245° C.

Beispiel G

N-Propyl-N′-[2,3-dihydro-3,3-dimethyl-2-oxo-(1H)indol-6-yl]-harnstoff

3.52 g (0.02 mol) 6-Amino-2,3-dihydro-3,3-dimethyl-(1H)indolin-2-on werden in 40 ml abs. Dioxan suspendiert und bei Raumtemperatur innerhalb von 10 min mit einer Lösung von 1.7 g (0.02 mol) n-Propylisocyanat in 10 ml Dioxan versetzt, worauf sich eine klare Lösung bildet. Kurz darauf beginnt sich das Produkt als kristalline Masse abzuscheiden. Nach 1 Stunde wird die Suspension mit Ligroin versetzt, der Niederschlag abgesaugt, mit Ligroin und Ether gewaschen und aus Ethanol umkristallisiert. Ausbeute 3.91 g (75 % d.Th.), Schmp. 223-225° C.

In analoger Weise wurden folgende Verbindungen hergestellt:

| | Verbindung | Schmelzpunkt [°C] |
|---|---|---|
| 1 | N-Methyl-N'-[2,3-dihydro-3,3-dimethyl-2-oxo-(1H)indol-6-yl]-harnstoff | 203 |
| 2 | N-Pentyl-N'-[2,3-dihydro-3,3-dimethyl-2-oxo-(1H)indol-6-yl]-harnstoff | 198-199 |
| 3 | N-Benzyl-N'-[2,3-dihydro-3,3-dimethyl-2-oxo-(1H)indol-6-yl]-harnstoff | 198 |
| 4 | N-(2-Phenylethyl)-N'-[2,3-dihydro-3,3-dimethyl-2-oxo-(1H)indol-6-yl]-harnstoff | 165-169 |
| 5 | N-(3-Phenylpropyl)-N'-[2,3-dihydro-3,3-dimethyl-2-oxo-(1H)indol-6-yl]-harnstoff | 152-154 |
| 6 | N-(4-Phenylbutyl)-N'-[2,3-dihydro-3,3-dimethyl-2-oxo-(1H)indol-6-yl]-harnstoff | 176-178 |
| 7 | N-Phenyl-N'-[2,3-dihydro-3,3-dimethyl-2-oxo-(1H)indol-6-yl]-harnstoff | 228-231 |
| 8 | N-(4-Methoxyphenyl)-N'-[2,3-dihydro-3,3-dimethyl-2-oxo-(1H)indol-6-yl]-harnstoff | 212-214 |
| 9 | N-(2-Methoxyphenyl)-N'-[2,3-dihydro-3,3-dimethyl-2-oxo-(1H)indol-6-yl]-harnstoff | 178 |
| 10 | N-(3,4-Dimethoxyphenyl)-N'-[2,3-dihydro-3,3-dimethyl-2-oxo-(1H)-indol-6-yl]-harnstoff | 210-214 |
| 11 | N-(3-Trifluormethylphenyl)-N'-[2,3-dihydro-3,3-dimethyl-2-oxo-(1H)indol-6-yl]-harnstoff | 212-214 |
| 12 | N-(4-Cyanphenyl)-N'-[2,3-dihydro-3,3-dimethyl-2-oxo-(1H)indol-6-yl]-harnstoff | 271-275 |

| | Verbindung | Schmelzpunkt [°C] |
|---|---|---|
| 13 | N-(2-Phenylethyl)-N'-[1',2'-dihydro-2'-oxospiro-(cyclopentan-1,3'-(3H)-indol)-6'-yl]harnstoff | 189-193 |
| 14 | N-Methyl-N'-[2,3-dihydro-3,3-dimethyl-2-oxo-(1H)indol-5-yl]-harnstoff | 233-235 |
| 15 | N-(4-Methylsulfonylphenyl)-N'-[2,3-dihydro-3,3-dimethyl-2-oxo-(1H)-indol-6-yl]harnstoff | 179-183 |
| 16 | N-[2-(4-Methoxyphenyl)ethyl]-N'-[1',2'-dihydro-2'-oxospiro-(cyclopentan-1,3'-(3H)-indol)-6'-yl]-harnstoff | 215-218 |
| 17 | N-Methyl-N'-[1,2,3,4-tetrahydro-4,4-dimethyl-2-oxochinolin-6-yl]harnstoff | 240-242 |
| 18 | N-Methyl-N'-[1,2,3,4-tetrahydro-4,4-dimethyl-2-oxochinolin-7-yl]harnstoff | 244-246 |
| 19 | N-(4-Chlorphenyl)-N'-[2,3-dihydro-3,3-dimethyl-2-oxo-(1H)-indol-6-yl]harnstoff | 242-244 |
| 20 | N-(4-Nitrophenyl)-N'-[2,3-dihydro-3,3-dimethyl-2-oxo-(1H)-indol-6-yl]harnstoff | 264-267 |
| 21 | N-(4-Aminophenyl)-N'-[2,3-dihydro-3,3-dimethyl-2-oxo-(1H)-indol-6-yl]harnstoff | 243-250 |
| 22 | N-(2-Allyloxyphenyl)-N'-[2,3-dihydro-3,3-dimethyl-2-oxo-(1H)-indol-6-yl]harnstoff | 205-206 |
| 23 | N-[4-(Methylsulfonyloxy)phenyl]-N'-[2,3-dihydro-3,3-dimethyl-2-oxo-(1H)-indol-6-yl]harnstoff | 245-247 |
| 24 | N-[3,4-(Methylendioxy)phenyl]-N'-[2,3-dihydro-3,3-dimethyl-2-oxo-(1H)-indol-6-yl]harnstoff | 223-225 |
| 25 | N-(4-Pyridyl)-N'-[2,3-dihydro-3,3-dimethyl-2-oxo-(1H)-indol-6-yl]harnstoff | 212-214 |

| | Verbindung | Schmelzpunkt [°C] |
|---|---|---|
| 26 | N-(3-Trifluormethylphenyl)-N'-[2,3-dihydro-3,3-dimethyl-2-oxo-(1H)-indol-5-yl]harnstoff | 193-194 |
| 27 | N-Phenyl-N'-[2,3-dihydro-3,3-dimethyl-2-oxo-(1H)-indol-5-yl]harn stoff | 253 |
| 28 | N-(3-Trifluormethylphenyl)-N'-[1',2'-dihydro-2'-oxospiro-(cyclopentan-1,3'-(3H)-indol)-6'-yl]-harnstoff | 230-232 |
| 29 | N-Phenyl-N'-[1',2'-dihydro-2'-oxospiro-(cyclopentan-1,3'-(3H)-indol)-6'-yl]harnstoff | 214-216 |
| 30 | N-(3-Trifluormethylphenyl)-N'-[1-ethyl-2,3-dihydro-3,3-dimethyl-2-oxoindol-6-yl]-harnstoff | 205-207 |
| 31 | N-(3-Chlorphenyl)-N'-[2,3-dihydro-3,3-dimethyl-2-oxo-(1H)-indol-6-yl]harnstoff | 227-228 |
| 32 | N-(3,4-Dichlorphenyl-N'-[2,3-dihydro-3,3-dimethyl-2-oxo-(1H)-indol-6-yl]harnstoff | 251-253 |
| 33 | N-(4-Methylphenyl)-N'-[2,3-dihydro-3,3-dimethyl-2-oxo-(1H)-indol-6-yl]harnstoff | 235-237 |
| 34 | N-(3-Methoxyphenyl)-N'-[2,3-dihydro-3,3-dimethyl-2-oxo-(1H)-indol-6-yl]harnstoff | 203-204 |
| 35 | N-Phenyl-N'-[1,2,3,4-tetrahydro-4,4-dimethyl-2-oxochinolin-7-yl]-harnstoff | 211-213 |
| 36 | N-(3-Trifluormethylphenyl)-N'-[1,2,3,4-tetrahydro-4,4-dimethyl-2-oxochinolin-7-yl]harnstoff | 221-222 |

Beispiel H

N-[2,3-Dihydro-3,3-dimethyl-2-oxo-(1H)indol-6-yl]carbamidsäure-hexylester

5.27 g (0.032 mol) Chlorameisensäurehexylester werden unter Eiskühlung innerhalb von 15 min zu einer Suspension aus 5.28 g (0.03 mol) 6-Amino-2,3-dihydro-3,3-dimethyl-(1H) indolin-2-on und 4.6 ml Triethylamin in 50 ml abs. Dichlormethan getropft. Dann wird die Lösung drei Stunden bei Raumtemperatur gerührt, das Lösungsmitel im Vakuum entfernt und der Rückstand durch Flash-Säulenchromatographie mit Heptan/2-Butanon 2/1 als Eluens gereinigt. Ausbeute 5.2 g (60 % d.Th.), Schmp. 180-183 °C nach

Umkristallisation aus Essigester.

## Ansprüche

1. Arzneimittel enthaltend mindestens eine Verbindung der allgemeinen Formel I

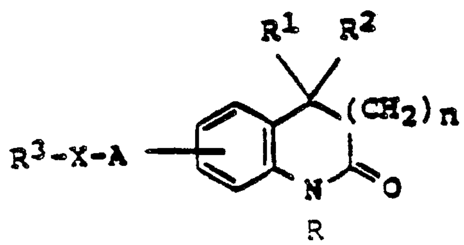

(I),

in welcher
R ein Wasserstoffatom, eine geradkettige oder verzweigte $C_1$-$C_6$-Alkylgruppe oder eine $C_3$-$C_6$-Cycloalkylgruppe darstellt,
$R^1$ ein Wasserstoffatom, eine $C_1$-$C_6$-Alkyl-, $C_2$-$C_6$-Alkenyl- oder eine $C_3$-$C_7$-Cycloalkylgruppe bedeutet,
$R^2$ eine $C_1$-$C_6$-Alkyl-, $C_2$-$C_6$-Alkenyl- oder Cyangruppe, eine durch eine Hydroxy-, $C_1$-$C_6$-Alkyl-, $C_1$-$C_6$-Alkoxy-, Amino-, $C_1$-$C_6$-Alkylamino-, Di-$C_1$-$C_6$-alkylamino- oder Hydrazinogruppe substituierte Carbonylgruppe bedeutet,
oder $R^1$ und $R^2$ zusammen eine $C_2$-$C_6$-Alkyliden- bzw. $C_3$-$C_6$-Cycloalkylidengruppe darstellen, oder $R^1$ und $R^2$ zusammen mit dem Kohlenstoffatom, an das sie gebunden sind, einen $C_3$-$C_7$-Spirocyclus bilden,
n gleich 0 oder 1 sein kann,
A die Gruppe -CO-NH-, -NH-CO-NH- oder -O-CO-NH-bedeutet, die über das Stickstoffatom an die Phenylgruppe gebunden ist,
X einen Valenzstrich, eine $C_1$-$C_4$-Alkylengruppe oder eine $C_2$-$C_4$-Alkenylengruppe darstellt,
$R^3$ einen Phenylring der allgemeinen Formel II

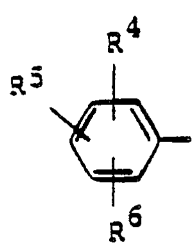

(II)

darstellt, wobei $R^4$, $R^5$, $R^6$ gleich oder verschieden sein können und jeweils Wasserstoff, eine $C_1$-$C_7$-Alkansulfonyloxy-, Trifluormethansulfonyloxy-, $C_1$-$C_7$-Alkansulfonylamino-, Trifluormethansulfonyl-amino-, N-$C_1$-$C_7$-Alkyl-$C_1$-$C_7$-alkansulfonylamino-, N-$C_1$-$C_7$-Alkyltrifluormethansulfonylamino-, $C_1$-$C_7$-Alkylsulfenylmethyl-, $C_1$-$C_7$-Alkylsulfinylmethyl- oder $C_1$-$C_7$-Alkylsulfonylmethylgruppe, eine durch eine Hydroxy-, Alkoxy-, Alkyl-, Amino-, $C_1$-$C_7$-Alkylamino-oder Di-$C_1$-$C_7$-alkylaminogruppe substituierte Carbonylgruppe, eine durch eine Amino-, $C_1$-$C_7$-Alkylamino-, Di-$C_1$-$C_7$-alkyalminogruppe, Morpholino-, Thiomorpholino-, Pyrrolidino-, Piperidino- oder Hexamethyleniminogruppe substituierte Sulfonylgruppe, eine $C_1$-$C_7$-Alkylcarbonylamino-, $C_1$-$C_7$-Alkylcarbonyloxy-, Aminocarbonylamino- oder $C_1$-$C_7$-Alkylaminocarbonylaminogruppe, eine $C_1$-$C_7$-Alkylmercapto-, $C_1$-$C_7$-Alkylsulfinyl- oder $C_1$-$C_7$-Alkylsulfonylgruppe, eine Nitro-, Amino-, Hydroxy-, Benzyloxy-, $C_1$-$C_7$-Alkoxy-, $C_1$-$C_7$-Alkyl-, $C_2$-$C_7$-Alkenyl-, $C_2$-$C_7$-Alkenyloxy-, $C_2$-$C_7$-Alkinyloxy-, Cyan-$C_1$-$C_7$-alkoxy-, Carboxy-$C_1$-$C_7$-alkoxy-, Phenyl $C_1$-$C_7$-alkoxy-, $C_1$-$C_7$-Alkoxy-carbonyl-$C_1$-$C_7$-alkoxy-, $C_1$-$C_7$-Alkylamino-, Di-$C_1$-$C_7$-alkylamino-, Trifluormethyl-, Cyano-, Halogen-oder Imidazolylgruppe sein können oder $R_3$ einen Methylendioxyphenylring bedeutet,
oder $R^3$ einen heterocyclischen Fünfring mit 1-4 Heteroatomen oder einen heterocyclischen Sechsring mit 1-5 Heteroatomen darstellt, wobei die Heteroatome gleich oder verschieden sein können und Sauerstoff, Stickstoff oder Schwefel bedeuten, und die heterocyclischen Fünf- oder Sechsring gewünschtenfalls an einem oder mehreren Stickstoffatomen ein Sauerstoffatom tragen können und gegebenenfalls durch eine

oder mehrere $C_1$-$C_6$-Alkyl-, $C_1$-$C_6$-Alkoxy-, $C_1$-$C_6$-Alkylmercapto-, Hydroxy-$C_1$-$C_6$-alkyl, Hydroxy-, Nitro-, Amino-, Halogen- oder Cyanogruppen substituiert sein können,
oder für den Fall, daß X eine Bindung darstellt, $R^3$ neben den oben genannten Gruppen auch eine $C_2$-$C_{12}$ Alkyl-, $C_3$-$C_7$-Cycloalkyl-, $C_2$-$C_6$-Alkenyl-, $C_1$-$C_6$-Alkoxy-$C_2$-$C_6$-alkenyl-, $C_3$-$C_7$-Cycloalkenyl-, $C_1$-$C_6$-Alkinyl-, Halogen-$C_1$-$C_6$-alkyl-, $C_1$-$C_6$-Alkoxy-$C_1$-$C_6$-alkyl-, Hydroxy-$C_1$-$C_6$-alkyl-, $C_4$-$C_6$-Alkandienyl-, Carboxy-$C_1$-$C_6$-alkyl-, $C_1$-$C_6$-Alkoxycarbonyl-$C_1$-$C_6$-alkyl- oder Formylamino-$C_1$-$C_6$-alkylgruppe bedeutet, wobei der Substituent $R^3$-X-A- in 4-, 5-, 6-, oder 7-Stellung mit dem 2,3-Dihydroindolin-2-on bzw. in 5-, 6-, 7- oder 8-Stellung mit dem 1,2,3,4-Tetrahydrochinolin-2-on verknüpft sein kann, oder
deren optisch aktive Formen, Tautomere oder physiologisch verträglichen Salze neben üblichen pharmazeutischen Hilfs-oder Trägerstoffen.

2. Arzneimittel enthaltend mindestens eine Verbindung der allgemeinen Formel I gemäß Anspruch 1, dadurch gekennzeichnet, daß
R ein Wasserstoffatom oder eine $C_1$-$C_3$-Alkylgruppe bedeutet,
$R^1$ ein Wasserstoffatom oder eine $C_1$-$C_6$-Alkylgruppe darstellt,
$R^2$ eine $C_1$-$C_6$-Alkylgruppe bedeutet oder $R_1$ und $R_2$ zusammen mit dem C-Atom, an das sie gebunden sind, einen $C_3$-$C_7$-Spirocyclus bilden,
X eine Bindung, eine $C_1$-$C_4$-Alkylenkette oder die Vinylengruppe darstellt, und
$R_3$ einen Phenylrest der allgemeinen Formel II bedeutet, in dem
$R_4$ Wasserstoff, eine $C_1$-$C_4$-Alkansulfonyloxy-, Trifluormethansulfonyloxy-, $C_1$-$C_4$-Alkansulfonylamino-, Trifluormethansulfonylamino-, N-$C_1$-$C_4$-Alkyl-$C_1$-$C_4$-alkansulfonylamino -, N-$C_1$-$C_4$-Alkyl-trifluormethansulfony lamino-, $C_1$-$C_4$-Alkylsulfinylmethyl-, $C_1$-$C_4$-Alkylsulfonylmethyl-, Aminocarbonyl-, Aminosulfonyl-, N-$C_1$-$C_4$-Alkyl-aminosulfonyl-, Di-$C_1$-$C_4$-alkylaminosulfonyl-, $C_1$-$C_4$-Alkylcarbonylamino-, $C_1$-$C_4$-Alkylmercapto-, $C_1$-$C_4$-Alkylsulfinyl-, $C_1$-$C_4$-Alkylsulfonyl-, Hydroxy-, $C_2$-$C_6$-Alkenyloxy-, $C_1$-$C_4$-Alkyl-, $C_1$-$C_4$-Alkoxy-, $C_2$-$C_6$-Alkinyloxy-, Cyan-$C_1$-$C_4$-alkoxy-, $C_1$-$C_4$-Alkoxycarbonyl-$C_1$-$C_4$-alkoxy-, Cyan-, Chlor-, Nitro-, Amino-, Di-$C_1$-$C_6$-alkylamino-, Trifluormethyl-oder 1-Imidazolylgruppe,
$R^5$ Wasserstoff, die $C_1$-$C_4$-Alkyl-, $C_1$-$C_4$-Alkoxy-, Hydroxy-, Di-$C_1$-$C_4$-alkyl-aminogruppe oder Chlor, und
$R^6$ Wasserstoff oder die $C_1$-$C_4$-Alkoxygruppe bedeutet, oder
$R^3$ den Pyrrol-, Furan-, Thiophen-, Pyrazol-, Imidazol-, Isothiazol-, Thiazol-, Oxazol-, Triazol-, Tetrazol-, Thiadiazol-, Isoxazol-, Oxadiazol-, Pyrazin-, Pyridazin-, Oxazin-, Thiazin-, Triazin-, Tetrazin-, Chinolin-, Pyridin-, N-Oxypyridin-, Pyrimidin- oder N,N'-Dioxypyrimidinrest darstellt, wobei diese Reste durch $C_1$-$C_6$-Alkyl-, $C_1$-$C_6$-Alkoxy, $C_1$-$C_6$-Alkylmercapto und Chlor substituiert sein können, oder $R_3$ einen Methylendioxyphenylring bedeutet,
oder
$R^3$ für den Fall, daß X eine Bindung darstellt, neben den oben genannten Gruppen auch eine $C_1$-$C_6$-Alkyl-, $C_2$-$C_6$-Alkenyl-, $C_4$-$C_6$-Alkandienyl-, $C_5$-$C_6$-Cycloalkenyl-, $C_5$-$C_6$-Cycloalkyl- oder $C_1$-$C_6$-Alkoxy$C_2$-$C_6$-alkenylgruppe bedeutet.

3. Arnzneimittel enthaltend eine der folgenden Verbindungen:
N-(3-Trifluormethylphenyl)-N'-[2,3-dihydro-3,3-dimethyl-2-oxo-(1H)-indol-6-yl]harnstoff
6-(3-Allyloxybenzoylamino)-2,3-dihydro-3,3-dimethyl-(1H)-indolin-2-on
6-[(2-Methoxy-4-methylsulfonyl)benzoylamino]-2,3-dihydro-3,3-dimethyl-(1H)-indolin-2-on
6-Hexadienoylamino-2,3-dihydro-3,3-dimethyl-(1H)-indolin-2-on
N-(2-Phenylethyl)-N'-[2,3-dihydro-3,3-dimethyl-2-oxo-(1H)-indol-6-yl]harnstoff
7-(4-Methoxybenzoylamino)-1,2,3,4-tetrahydro-4,4-dimethylchinolin-2-on

4. Arzneimittel enthaltend Verbindungen der allgemeinen Formel I gemäß Anspruch 1 oder 2, dadurch gekennzeichnet, daß
n die Zahl 0 bedeutet,
A die Gruppe -CO-NH- darstellt und
$R_3$ einen Phenylring der allgemeinen Formel II oder einen heterocyclischen Fünf- oder Sechsring wie in Anspruch 1 oder 2 angegeben bedeutet.

5. Verbindungen der allgemeinen Formel I

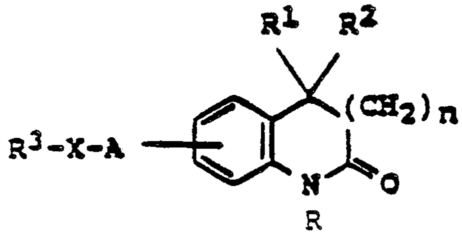

(I),

in der
R ein Wasserstoffatom, eine geradkettige oder verzweigte $C_1$-$C_6$-Alkylgruppe oder eine $C_3$-$C_6$-Cycloalkylgruppe darstellt,
$R^1$ ein Wasserstoffatom, eine $C_1$-$C_6$-Alkyl-, $C_2$-$C_6$-Alkenyl- oder eine $C_3$-$C_7$-Cycloalkylgruppe bedeutet,
$R^2$ eine $C_1$-$C_6$-Alkyl-, $C_2$-$C_6$-Alkenyl- oder Cyangruppe, eine durch eine Hydroxy-, $C_1$-$C_6$-Alkyl-, $C_1$-$C_6$-Alkoxy-, Amino-, $C_1$-$C_6$-Alkylamino-, Di-$C_1$-$C_6$-alkylamino- oder Hydrazinogruppe substituierte Carbonylgruppe bedeutet,
oder $R^1$ und $R^2$ zusammen eine $C_2$-$C_6$-Alkyliden- bzw. $C_3$-$C_6$-Cycloalkylidengruppe darstellen, oder $R^1$ und $R^2$ zusammen mit dem Kohlenstoffatom, an das sie gebunden sind, einen $C_3$-$C_7$-Spirocyclus bilden,
n die Zahlen 0 oder 1 bedeutet,
A die Gruppe -CO-NH-, -NH-CO-NH- oder -O-CO-NH-bedeutet, die über das Stickstoffatom an die Phenylgruppe gebunden ist,
X einen Valenzstrich, eine $C_1$-$C_4$-Alkylengruppe oder eine $C_2$-$C_4$-Alkenylengruppe darstellt,
$R^3$ einen Phenylring der allgemeinen Formel II

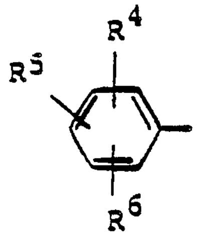

(II)

darstellt, wobei $R^4$, $R^5$, $R^6$ gleich oder verschieden sein können und jeweils Wasserstoff, eine $C_1$-$C_7$-Alkansulfonyloxy-, Trifluormethansulfonyloxy-, $C_1$-$C_7$-Alkansulfonylamino-, Trifluormethansulfonylamino-, N-$C_1$-$C_7$-Alkyl-$C_1$-$C_7$-alkansulfonylamino-, N- $C_1$-$C_7$-Alkyltrifluormethansulfonylamino-, $C_1$-$C_7$-Alkylsulfenylmethyl-, $C_1$-$C_7$-Alkylsulfinylmethyl- oder $C_1$-$C_7$-Alkylsulfonylmethylgruppe, eine durch eine Hydroxy-, Alkoxy-, Alkyl-, Amino-, $C_1$-$C_7$-Alkylamino-oder Di-$C_1$-$C_7$-alkylaminogruppe substituierte Carbonylgruppe, eine durch eine Amino-, $C_1$-$C_7$-Alkylamino-, Di-$C_1$-$C_7$-alkylamino-, Morpholino-, Thiomorpholino-, Pyrrolidino-, Piperidino- oder Hexamethyleniminogruppe substituierte Sulfonylgruppe eine $C_1$-$C_7$-Alkylcarbonylamino-, $C_1$-$C_7$-Alkylcarbonyloxy-, Aminocarbonylamino- oder $C_1$-$C_7$-Alkylaminocarbonylaminogruppe, eine $C_1$-$C_7$-Alkylmercapto-, $C_1$-$C_7$-Alkylsulfinyl- oder $C_1$-$C_7$-Alkylsulfonylgruppe, eine Nitro-, Amino-, Hydroxy-, Benzyloxy-, $C_1$-$C_7$-Alkenyl, $C_1$-$C_7$-Alkoxy-, $C_1$-$C_7$-Alkyl-, $C_2$-$C_7$-Alkenyl-, $C_2$-$C_7$-Alkenyloxy-, $C_2$-$C_7$-Alkinyloxy-, Cyan-$C_1$-$C_7$-alkoxy-, Carboxy-$C_1$-$C_7$- alkoxy-, Phenyl-$C_1$-$C_7$-alkoxy-, $C_1$-$C_7$-Alkoxycarbonyl-$C_1$-$C_7$-alkoxy-, $C_1$-$C_7$-Alkylamino-, Di-$C_1$-$C_7$-alkylamino-, Trifluormethyl-, Cyano-, Halogen- oder Imidazolylgruppe sein können oder $R_3$ einen Methylendioxyphenylring bedeutet,
oder $R^3$ einen heterocyclischen Fünfring mit 1-4 Heteroatomen oder einen heterocyclischen Sechsring mit 1-5 Heteroatomen darstellt, wobei die Heteroatome gleich oder verschieden sein können und Sauerstoff, Stickstoff oder Schwefel bedeuten, und die heterocyclischen Fünf- oder Sechsring gewünschtenfalls an einem oder mehreren Stickstoffatomen ein Sauerstoffatom tragen können und gegebenenfalls durch eine oder mehrere $C_1$-$C_6$-Alkyl-, $C_1$-$C_6$-Alkoxy-, $C_1$-$C_6$-Alkylmercapto-, Hydroxy-$C_1$-$C_6$-alkyl, Hydroxy-, Nitro-, Amino-, Halogen- oder Cyanogruppen substituiert sein können,
oder für den Fall, daß X eine Bindung darstellt, $R^3$ neben den oben genannten Gruppen auch eine $C_2$-$C_{12}$ Alkyl-, $C_3$-$C_7$-Cycloalkyl-, $C_2$-$C_6$-Alkenyl-, $C_1$-$C_6$-Alkoxy-$C_2$-$C_6$-alkenyl-, $C_3$-$C_7$-Cycloalkenyl-, $C_1$-$C_6$-Alkinyl-, Halogen-$C_1$-$C_6$-alkyl-, $C_1$-$C_6$-Alkoxy-$C_1$-$C_6$-alkyl-, Hydroxy-$C_1$-$C_6$-alkyl-, $C_4$-$C_6$-Alkandienyl-, Carboxy-$C_1$-$C_6$-alkyl-, $C_1$-$C_6$-Alkoxycarbonyl-$C_1$-$C_6$-alkyl- oder Formylamino-$C_1$-$C_6$-alkylgruppe bedeutet, wobei der Substituent $R^3$-X-A- in 4-, 5-, 6-, oder 7-Stellung mit dem 2,3-Dihydroindolin-2-on bzw. in 5-, 6-, 7- oder 8-Stellung

mit dem 1,2,3,4-Tetrahydrochinolin-2-on verknüpft sein kann, oder deren optisch aktive Formen, Tautomere oder physiologisch verträglichen Salze, mit der Maßgabe, daß für den Fall, daß n die Zahl 0 und A die Gruppe -CO-NH- bedeutet, $R_3$ nicht einen Phenylring der allgemeinen Formel II oder einen heterocyclischen Fünf- oder Sechsring bedeuten kann.

6. Verbindungen der allgemeinen Formel I gemäß Anspruch 5, in der
R ein Wasserstoffatom oder eine $C_1$-$C_3$-Alkylgruppe bedeutet,
$R^1$ ein Wasserstoffatom oder eine $C_1$-$C_6$-Alkylgruppe darstellt,
$R^2$ eine $C_1$-$C_6$-Alkylgruppe bedeutet oder $R_1$ und $R_2$ zusammen mit dem C-Atom, an das sie gebunden sind, einen $C_3$-$C_7$-Spirocyclus bilden,
X eine Bindung, eine $C_1$-$C_4$-Alkylenkette oder die Vinylengruppe darstellt, und
$R_3$ einen Phenylrest der allgemeinen Formel II bedeutet, in dem
$R_4$ Wasserstoff, eine $C_1$-$C_4$-Alkansulfonyloxy-, Trifluormethansulfonyloxy-, $C_1$-$C_4$-Alkansulfonylamino-, Trifluormethansulfonylamino-, N-$C_1$-$C_4$-Alkyl-$C_1$-$C_4$-alkansulfonylamino -, N-$C_1$-$C_4$-Alkyl-trifluormethansulfony lamino-, $C_1$-$C_4$-Alkylsulfinylmethyl-, $C_1$-$C_4$-Alkylsulfonylmethyl-, Aminocarbonyl-, Aminosulfonyl-, N-$C_1$-$C_4$-Alkyl-aminosulfonyl-, Di-$C_1$-$C_4$-alkylaminosulfonyl-, $C_1$-$C_4$-Alkylcarbonylamino-, $C_1$-$C_4$-Alkylmercapto-, $C_1$-$C_4$-Alkylsulfinyl-, $C_1$-$C_4$-Alkylsulfonyl-, Hydroxy-, $C_2$-$C_6$-Alkenyloxy-, $C_1$-$C_4$-Alkyl-, $C_1$-$C_4$-Alkoxy-, $C_2$-$C_6$-Alkinyloxy-, Cyan-$C_1$-$C_4$-alkoxy-, $C_1$-$C_4$-Alkoxycarbonyl-$C_1$-$C_4$-alkoxy-, Cyan-, Chlor-, Nitro-, Amino-, Di-$C_1$-$C_4$-alkylamino-, Trifluormethyl-oder 1-Imidazolylgruppe,
$R^5$ Wasserstoff, die $C_1$-$C_4$-Alkyl-, $C_1$-$C_4$-Alkoxy-, Hydroxy-, Di-$C_1$-$C_4$-alkyl-aminogruppe oder Chlor, und
$R_6$ Wasserstoff oder die $C_1$-$C_4$-Alkoxygruppe bedeutet, oder
$R^3$ den Pyrrol-, Furan-, Thiophen-, Pyrazol-, Imidazol-, Isothiazol-, Thiazol-, Oxazol-, Triazol-, Tetrazol-, Thiadiazol-, Isoxazol-, Oxadiazol-, Pyrazin-, Pyridazin-, Oxazin-, Thiazin-, Triazin-, Tetrazin-, Chinolin-, Pyridin-, N-Oxypyridin-, Pyrimidin- und N,N-Dioxypyrimidinrest darstellt, wobei diese Reste durch $C_1$-$C_6$-Alkyl-, $C_1$-$C_6$-Alkoxy, $C_1$-$C_6$-Alkylmercapto und Chlor substituiert sein können, oder $R_3$ einen Methylendioxyphenylring bedeutet,
oder
$R^3$ für den Fall, daß X eine Bindung darstellt, neben den oben genannten Gruppen auch eine $C_1$-$C_6$-Alkyl-, $C_2$-$C_6$-Alkenyl-, $C_4$-$C_6$-Alkandienyl-, $C_5$-$C_6$-Cycloalkenyl-, $C_5$-$C_6$-Cycloalkyl- oder $C_1$-$C_6$-Alkoxy-$C_2$-$C_6$-alkenylgruppe bedeutet.

7. Verwendung von Verbindungen gemäß Anspruch 5 oder 6 zur Herstellung von Arzneimitteln.

8. Verfahren zur Herstellung von Lactamen der allgemeinen Formel I

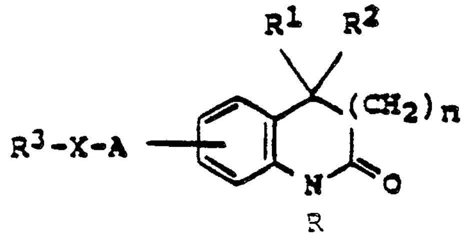
(I),

in welcher
R ein Wasserstoffatom, eine geradkettige oder verzweigte $C_1$-$C_6$-Alkylgruppe oder eine $C_3$-$C_6$-Cycloalkylgruppe darstellt,
$R^1$ ein Wasserstoffatom, eine $C_1$-$C_6$-Alkyl-, $C_2$-$C_6$-Alkenyl- oder eine $C_3$-$C_7$-Cycloalkylgruppe bedeutet,
$R^2$ eine $C_1$-$C_6$-Alkyl-, $C_2$-$C_6$-Alkenyl- oder Cyangruppe, eine durch eine Hydroxy-, $C_1$-$C_6$-Alkyl-, $C_1$-$C_6$-Alkoxy-, Amino-, $C_1$-$C_6$-Alkylamino-, Di-$C_1$-$C_6$-alkylamino- oder Hydrazinogruppe substituierte Carbonylgruppe bedeutet,
oder $R^1$ und $R^2$ zusammen eine $C_2$-$C_6$-Alkyliden- bzw. $C_3$-$C_6$-Cycloalkylidengruppe darstellen, oder $R^1$ und $R^2$ zusammen mit dem Kohlenstoffatom, an das sie gebunden sind, einen $C_3$-$C_7$-Spirocyclus bilden,
n die Zahlen 0 oder 1 bedeutet,
A die Gruppe -CO-NH-, -NH-CO-NH- oder -O-CO-NH-bedeutet, die über das Stickstoffatom an die Phenylgruppe gebunden ist,
X einen Valenzstrich, eine $C_1$-$C_4$-Alkylenkette oder eine $C_2$-$C_4$-Alkenylengruppe darstellt,
$R^3$ einen Phenylring der allgemeinen Formel II

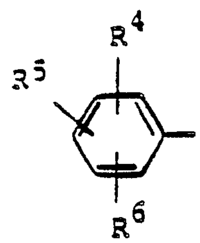

(II)

darstellt, wobei $R^4$, $R^5$, $R^6$ gleich oder verschieden sein können und jeweils Wasserstoff, eine $C_1$-$C_7$-Alkansulfonyloxy-, Trifluormethansulfonyloxy-, $C_1$-$C_7$-Alkansulfonylamino-, Trifluormethansulfonylamino-, N-$C_1$-$C_7$-Alkyl-$C_1$-$C_7$-alkansulfonylamino-, N-$C_1$-$C_7$-Alkyltrifluormethansulfonylamino-, $C_1$-$C_7$-Alkylsulfenylmethyl-, $C_1$-$C_7$-Alkylsulfinylmethyl- oder $C_1$-$C_7$-Alkylsulfonylmethylgruppe, eine durch eine Hydroxy-, Alkoxy-, Alkyl-, Amino-, $C_1$-$C_7$-Alkylamino-oder Di-$C_1$-$C_7$-alkylaminogruppe substituierte Carbonylgruppe, eine durch eine Amino-, $C_1$-$C_7$-Alkylamino-, Di-$C_1$-$C_7$-alkylamino, Morpholino-, Thiomorpholino-, Pyrrolidino-, Piperidino- oder Hexamethyleniminogruppe substituierte Sulfonylgruppe, eine $C_1$-$C_7$-Alkylcarbonylamino-, $C_1$-$C_7$-Alkylcarbonyloxy-, Aminocarbonylamino- oder $C_1$-$C_7$-Alkylaminocarbonylaminogruppe, eine $C_1$-$C_7$-Alkylmercapto-, $C_1$-$C_7$-Alkylsulfinyl- oder $C_1$-$C_7$-Alkylsulfonylgruppe, eine Nitro-, Amino-, Hydroxy-, Benzyloxy-, $C_1$-$C_7$-Alkoxy-, $C_1$-$C_7$-Alkyl-, $C_2$-$C_7$-Alkenyl-, $C_2$-$C_7$-Alkenyloxy-, $C_2$-$C_7$-Alkinyloxy-, Cyan-$C_1$-$C_7$-alkoxy-, Carboxy-$C_1$-$C_7$-alkoxy-, Phenyl-$C_1$-$C_7$-alkoxy-, $C_1$-$C_7$-Alkoxycarbonyl-$C_1$-$C_7$-alkoxy-, $C_1$-$C_7$-Alkylamino-, Di-$C_1$-$C_7$-alkylamino-, Trifluormethyl-, Cyano-, Halogen- oder Imidazolylgruppe sein können oder $R_3$ einen Methylendioxyphenylring bedeutet,

oder $R^3$ einen heterocyclischen Fünfring mit 1-4 Heteroatomen oder einen heterocyclischen Sechsring mit 1-5 Heteroatomen darstellt, wobei die Heteroatome gleich oder verschieden sein können und Sauerstoff, Stickstoff oder Schwefel bedeuten, und die heterocyclischen Fünf- oder Sechsring gewünschtenfalls an einem oder mehreren Stickstoffatomen ein Sauerstoffatom tragen können und gegebenenfalls durch eine oder mehrere $C_1$-$C_6$-Alkyl-, $C_1$-$C_6$-Alkoxy-, $C_1$-$C_6$-Alkylmercapto-, Hydroxy-$C_1$-$C_6$-alkyl, Hydroxy-, Nitro-, Amino-, Halogen- oder Cyanogruppen substituiert sein können,

oder für den Fall, daß X eine Bindung darstellt, $R^3$ neben den oben genannten Gruppen auch eine $C_2$-$C_{12}$ Alkyl-, $C_3$-$C_7$-Cycloalkyl-, $C_2$-$C_6$-Alkenyl-, $C_1$-$C_6$-Alkoxy-$C_2$-$C_6$-alkenyl-, $C_3$-$C_7$-Cycloalkenyl-, $C_1$-$C_6$-Alkinyl-, Halogen-$C_1$-$C_6$-alkyl-, $C_1$-$C_6$-Alkoxy-$C_1$-$C_6$- alkyl-, Hydroxy-$C_1$-$C_6$-alkyl-, $C_3$-$C_6$-Alkandienyl-, Carboxy-$C_1$-$C_6$-alkyl-, $C_1$-$C_6$-Alkoxycarbonyl-$C_1$-$C_6$-alkyl- oder Formylamino-$C_1$-$C_6$-alkylgruppe bedeutet, wobei der Substituent $R^3$-X-A- in 4-, 5-, 6-, oder 7-Stellung mit dem 3,4-Dihydroindolin-2-on bzw. in 5-, 6-, 7- oder 8-Stellung mit dem 1,2,3,4-Tetrahydrochinolin-2-on verknüpft sein kann, oder

deren optisch aktive Formen, Tautomere oder physiologisch verträglichen Salze, mit der Maßgabe, daß für den Fall, daß n die Zahl 0 und A die Gruppe -CO-NH- bedeutet, $R_3$ nicht einen Phenylring der allgemeinen Formel II oder einen heterocyclischen Fünf- oder Sechsring bedeuten kann,

dadurch gekennzeichnet, daß man in an sich bekannter Weise ein Amin der allgemeinen Formel XV

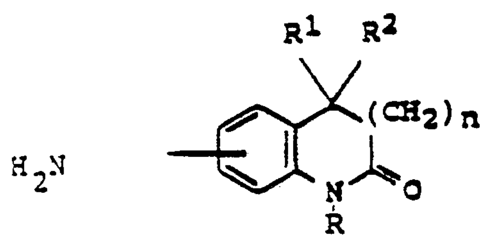

(XV)

in der R, $R_1$, $R_2$ und n die angegebene Bedeutung haben entweder

a) mit einer Verbindung der allgemeinen Formel VII

$R^3$-X-CO-Y (VII),

in der $R^3$ und X die oben angegebene Bedeutung haben und Y einen leicht abspaltbaren Rest, wie ein Halogenatom, eine Methoxy- oder Ethoxygruppe bzw. die Verbindung $R^3$-X-CO-Y ein Anhydrid oder ein anderes aktiviertes Carbonsäurederivat darstellt, umsetzt,

oder

b) mit einem Isocyanat der Formel VIII

$R^3$-X-N=C=O (VIII),

in der $R^3$ und X die oben angegebene Bedeutung haben, umsetzt,
oder
c) mit einer Verbindung der Formel IX
$R^3$-X-O-CO-Y (IX),
in der $R^3$, X und Y die oben angegebene Bedeutung haben, umsetzt
und eine erfindungsgemäß erhaltene Verbindung der allgemeinen Formel I gewünschtenfalls in ein anderes Derivat der Formel I oder dessen Tautomer umwandelt und/oder eine erhaltene Verbindung der allgemeinen Formel I, dessen Tautomer, Enantiomer bzw. Stereoisomer in ein physiologisch verträgliches Salz überführt.

9. Verfahren gemäß Anspruch 8 zur Herstellung von Lactamen der allgemeinen Formel I
in der
R ein Wasserstoffatom oder eine $C_1$-$C_3$-Alkylgruppe bedeutet,
$R^1$ ein Wasserstoffatom oder eine $C_1$-$C_6$-Alkylgruppe darstellt,
$R^2$ eine $C_1$-$C_6$-Alkylgruppe bedeutet oder $R_1$ und $R_2$ zusammen mit dem C-Atom, an das sie gebunden sind, einen $C_3$-$C_7$-Spirocyclus bilden,
X eine Bindung, eine $C_1$-$C_4$-Alkylenkette oder die Vinylengruppe darstellt, und
$R_3$ einen Phenylrest der allgemeinen Formel II bedeutet, in dem
$R_4$ Wasserstoff, eine $C_1$-$C_4$-Alkansulfonyloxy-, Trifluormethansulfonyloxy-, $C_1$-$C_4$-Alkansulfonylamino-, Trifluormethansulfonylamino-, N-$C_1$-$C_4$-Alkyl-$C_1$-$C_4$-alkansulfonylamino-, N-$C_1$-$C_4$-Alkyl-trifluormethansulfon ylamino-, $C_1$-$C_4$-Alkylsulfinylmethyl-, $C_1$-$C_4$-Alkylsulfonylmethyl-, Aminocarbonyl-, Aminosulfonyl-, N-$C_1$-$C_4$-Alkyl-aminosulfonyl-, Di-$C_1$-$C_4$-alkylaminosulfonyl-, $C_1$-$C_4$-Alkylcarbonylamino-, $C_1$-$C_4$-Alkylmercapto-, $C_1$-$C_4$-Alkylsulfinyl-, $C_1$-$C_4$-Alkylsulfonyl-, Hydroxy-, Benzyloxy-, $C_2$-$C_6$-Alkenyloxy-, $C_1$-$C_4$-Alkyl-, $C_1$-$C_4$-Alkoxy-, $C_2$-$C_6$-Alkenyloxy-, Cyan-$C_1$-$C_4$-alkoxy-, $C_1$-$C_4$-Alkoxy-carbonyl-$C_1$-$C_4$-alkoxy-, Cyan-, Chlor-, Nitro-, Amino-, Di-$C_1$-$C_4$-alkylamino-, Trifluormethyl- oder 1-Imidazolylgruppe,
$R^5$ Wasserstoff, die $C_1$-$C_4$-Alkyl-, $C_1$-$C_4$-Alkoxy-, Hydroxy-, Di-$C_1$-$C_4$-alkyl-aminogruppe oder Chlor, und
$R^6$ Wasserstoff oder die $C_1$-$C_4$-Alkoxygruppe bedeutet, oder
$R^3$ den Pyrrol-, Furan-, Thiophen-, Pyrazol-, Imidazol-, Isothiazol-, Thiazol-, Oxazol-, Triazol-, Tetrazol-, Thiadiazol-, Isoxazol-, Oxadiazol-, Pyrazin-, Pyridazin-, Oxazin-, Thiazin-, Triazin-, Tetrazin-, Chinolin-, Pyridin-, N-Oxypyridin-, Pyrimidin- und N,N-Dioxypyrimidinrest darstellt, wobei diese Reste durch $C_1$-$C_6$-Alkyl-, $C_1$-$C_6$-Alkoxy, $C_1$-$C_6$-Alkylmercapto und Chlor substituiert sein können, oder $R_3$ einen Methylendioxyphenylring bedeutet,
oder
$R^3$ für den Fall, daß X eine Bindung darstellt, neben den oben genannten Gruppen auch eine $C_1$-$C_6$-Alkyl-, $C_2$-$C_6$-Alkenyl-, $C_4$-$C_6$-Alkandienyl-, $C_5$-$C_6$-Cycloalkenyl-, $C_5$-$C_6$-Cycloalkyl- oder $C_1$-$C_6$-Alkoxy-$C_2$-$C_6$-alkenylgruppe bedeutet.

10. Verwendung von Verbindungen der allgemeinen Formel I gemäß einem der Ansprüche 1-4 zur Herstellung von Arzneimitteln mit erythrozytenaggregations- und/oder thrombozytenaggregationshemmender Wirkung.

11. Arzneimittel gemäß einem der Ansprüche 1 bis 4 mit erythrozytenaggregations- oder thrombozytenaggregationshemmender Wirkung.

12. Arzneimittel enthaltend mindestens eine Verbindung gemäß Anspruch 5 oder 6 oder deren nicht toxischen Salze neben üblichen pharmakologischen Träger- oder Hilfsstoffen.